# EUROPEAN PATENT APPLICATION

(11) **EP 2 277 513 A2**
(43) Date of publication of application: **26.01.2011**
(21) Application number: 10177541.9
(22) Date of filing: 21.04.2004
(51) Int. Cl.: A61K 31/00, A61K 31/495, A61P 13/10

(54) **Treatment of incontinence with 5htc2 agonists**

(30) Priority: 25.04.2003 GB 0309533
(62) Divisional of application: 04728610.9
(71) Applicant: Pfizer Inc., New York, NY 10017 (US); Pfizer Limited, Sandwich, Kent CT13 9NJ (GB)
(72) Inventor: McMurray, Gordon, Sandwich, Kent CT13 9NJ (GB); Miner, Wesley, Dennis, Sandwich, Kent CT13 9NJ (GB)
(74) Representative: England, Peter Michael

(57) **Abstract**

The present invention relates to the use of agonists of 5-HT2C receptors for the treatment of stress urinary incontinence. The present invention also relates to a method of treatment of stress urinary incontinence, to assays to screen for compounds useful in the treatment of stress urinary incontinence, and to methods of preparing compositions for the treatment of stress urinary incontinence.

## Description

### FIELD OF INVENTION

The present invention relates to the use of agonists of 5-HT2C receptors for the treatment of incontinence, preferably mixed incontinence or stress urinary incontinence. The invention also relates to the use of antagonists of 5-HT2C receptors for the treatment of urine retention or detrusor sphincter dyssynergia.

The present invention also relates to a method of treatment of incontinence.

The present invention also relates to assays to screen for compounds useful in the treatment of incontinence.

### Introduction

Urinary incontinence is the complaint of any involuntary leakage of urine. It is a common condition, and often constitutes an embarrassment which can lead to social isolation, depression, loss of quality of life, and is a major cause for institutionalisation in the elderly population.

The medical need is high for effective pharmacological treatments of mixed incontinence and stress urinary incontinence (SUI). This high medical need is a result of lack of efficacious pharmacological therapy coupled with high patient numbers. Recent estimates put the number of people suffering from SUI in the USA at 18 million, with women predominantly affected.

It is increasingly recognised that both supraspinal and spinal sites contain key neuroanatomical areas involved in the control of urethral sphincter mechanisms, such as external urethral sphincter (EUS) tone, particularly during the initiation of the "guarding reflex" which sustains continence during abrupt increases in abdominal pressure (e.g. cough, sneeze, laugh).

Additionally, the neurotransmitter serotonin (5-HT) has a key role in mechanisms involved in micturition and continence.

Receptors for serotonin are an important class of G protein-coupled receptors. Serotonin is thought to play a role in processes related to learning and memory, sleep, thermoregulation, mood, motor activity, pain, sexual and aggressive behaviours, appetite, neurodegenerative regulation, and biological rhythms. Serotonin receptors are currently classified into seven subfamilies (5-HT1 through to 5-HT7; Hoyer, D. et al (1994) Pharmacol. Rev. 46, 157-203); several of the subfamilies are further divided into subtypes. For example, the 5-HT2 subfamily is currently divided into three subtypes, 5-HT2A, 5-HT2B, and 5-HT2C. All three 5-HT2 subtypes are linked to phospholipase C with the generation of two second messengers, diacylglycerol (which activates protein kinase C) and inositol trisphosphate (which releases intracellular stores of Ca²⁺).

The 5-HT2C receptor was first cloned from rat (then named 5-HT1 C: Julius, D. et al (1988) Science 241, 558-564); the human 5-HT2C receptor was cloned by Saltzman, A.G. et al ((1991) Biochem. Biophys. Res. Commun. 181, 1469-1478), and the sequence can be found in SwissProt Accession number P28335.

Several serotonin receptors have been suggested as therapeutic targets for incontinence, e.g. 5-HT1A receptors (WO 97/31637), 5-HT3 receptors (EP 467365), 5-HT7 receptors (WO 00/69437) or 5-HT1F receptors (US 2003004207). Indeed evidence exists to suggest that compounds with agonist activity at 5-HT2C receptors may be beneficial in the treatment of conditions related to abnormal bladder activity. In particular, in a rat model evaluating the effect of 5-HT receptor agonists on volume induced rhythmic bladder contractions in anaesthetised rats, compounds with 5-HT2C agonist activity were shown to abolish such reflexly evoked bladder contractions, although in conscious animals these agonists were concluded not to have any effect on the frequency of voiding (Steers W.D. et al. (1989) Am. J. Physiol. 257, R1441-R1449; Steers W.D. et al. (1992) Drug Dev. Res. 27, 361-375; Guarneri L. et al. (1996) Neurourol. Urodynam. 15, 316-317). Interestingly in one of these studies, which also looked at the effect of 5-HT2C agonists in anaesthetised rats during normal bladder filling the authors concluded that such agonists had no effect on normal micturition parameters (Steers W.D. et al. (1982) Drug Dev. Res. 27, 361-375). More recently, a compound later shown to be an agonist at 5-HT2 receptors, including the 5-HT2C subtype (Leysen,D.C.M (1999) IDrugs 2, 109-120), 1-[6-chloro-5-(trifluoromethyl)-2-pyridinyl]piperazine (Org-12962) has been shown to reduce urine wet spot size after subcutaneous administration in mice, although this was only significant at the highest dose tested (WO 98/33504). Such a finding would suggest a reduction in the actual volume voided and hence a similar effect in reducing bladder contraction as that suggested above. Surprisingly, we have now found that a 5-HT2C receptor agonist is useful for treating incontinence, especially stress urinary incontinence and mixed urinary incontinence due to effects on the urethra.

### Aspects of the Invention

A seminal finding of the present invention is the ability to treat incontinence, especially mixed incontinence and stress urinary incontinence, with an agonist for 5-HT2C receptors.

Therefore, the invention relates to 5-HT2C receptor agonists (provided the agonist is not 1-[6-chloro-5-(trifluoromethyl)-2-pyridinyl]piperazine (Org-12962)) for use in the treatment of incontinence, preferably urinary incontinence, even more preferably mixed incontinence and stress urinary incontinence. The invention also relates to the use of 5-HT2C receptor agonists (provided the agonist is not 1-[6-chloro-5-(trifluoromethyl)-2-pyridinyl]piperazine (Org-12962)) for the manufacture of a medicament for the treatment of incontinence. The invention also relates to a method of treatment of incontinence with 5-HT2C receptor agonists (provided the agonist is not 1-[6-chloro-5-(trifluoromethyl)-2-pyridinyl]piperazine (Org-12962)). One aspect of the invention is therefore a method of treating incontinence, comprising the administration to a patient in need of such treatment of an effective amount of a 5-HT2C receptor agonist (provided the agonist is not 1-[6-chloro-5-(trifluoromethyl)-2-pyridinyl]piperazine (Org-12962)). The term "incontinence" includes any disorder which involves any involuntary leakage of urine. Examples of different forms of incontinence are stress urinary incontinence, which is the complaint of involuntary leakage of urine on effort or exertion, or on sneezing or coughing; and mixed (urinary) incontinence, which is the complaint of involuntary leakage associated with urgency and also with exertion, effort, sneezing or coughing, urgency referring to a sudden, compelling desire to pass urine which is difficult to defer. The term "treating incontinence" includes the palliative, curative and prophylactic treatment of incontinence, complications arising from incontinence such as depression, social isolation, and institutionalisation.

Another aspect of the invention is the use of a 5-HT2C antagonist for the treatment of urine retention and detrusor sphincter dyssynergia, as the antagonist reduces urethral tone. Patients suffering from urine retention include, for example, men with benign prostatic hyperplasia (BPH), or people with spinal cord injuries. Yet another aspect of the invention is a method of treatment of urine retention using a 5-HT2C antagonist.

The 5-HT2C receptor agonist preferably will have an EC₅₀ in a functional assay measuring 5-HT2C receptor activation of less than 1 µM, more preferably an EC₅₀ of less than 100nM, more preferably an EC₅₀ of less than 10nM, even more preferably an EC₅₀ of less than 1nM. The EC₅₀ may be measured in a functional assay, e.g. measuring rise of intracellular calcium upon agonist stimulation, e.g. using fluorescent dyes such as Fluo-3 or Fluo-4 (see, for example, Example 3 herein).

Preferably the 5-HT2C receptor agonists will be at least 10 fold selective over other 5-HT receptors, more preferably at least 100 fold selective over other 5-HT receptors. Preferably the 5-HT2C receptor agonists will be at least 10 fold selective over adrenergic receptors, more preferably at least 100 fold selective over adrenergic receptors. More preferably, the 5-HT2C receptor agonists will be at least 10 fold selective over other 5-HT receptors and at least 10 fold selective over adrenergic receptors, most preferably at least 100 fold selective over other 5-HT receptors and at least 100 fold selective over adrenergic receptors.

One embodiment of the invention is the use of m-CPP in the manufacture of a medicament for the treatment of incontinence. Suitable 5-HT2C receptor agonists for use in the invention also include a pharmaceutically acceptable salt of m-CPP (m-chlorophenylpiperazine - commercially available from Sigma Aldrich Product number C-5554); PNU-22394A, nordexfenfluramine, MK-212 (Tocris, Cat No 0941), WAY-161503 (Tocris, Cat No 1801), YM-348, or pharmaceutically acceptable salts thereof.

Preferred suitable 5-HT2C receptor agonists for use in the invention include:
(*RS*)-1-(7-Methylthio-2,3-dihydro-1*H*-pyrrolo[1,2-*a*]indol-9-yl)-2-propylamine (Example 11 in WO 00/12510),
1-(7-chloro-2,3-dihydro-1*H*-pyrrolo[1,2-*a*]indol-9-yl-2-propylamine (WO 00/12510),
1-(6,7-difluoro-2,3-dihydro-1*H*-pyrrolo[1,2-*a*]indol-9-yl-2-propylamine (WO 00/12510),
1-(7-bromo-2,3-dihydro-1*H*-pyrrolo[1,2-*a*]indol-9-yl-2-propylamine (WO 00/12510),
1-(7-methoxy-2,3-dihydro-1*H*-pyrrolo[1,2-*a*]indol-9-yl-2-propylamine (WO 00/12510);
(S)-2-(6-ethoxy-2,3-dihydro-1H-3a-aza-cyclopenta[a]inden-8-yl)-1-methyl-ethylamine (WO 02/051844),
(S)-2-[6-(2-methoxy-ethoxy)- 2,3-dihydro-1H-3a-aza-cyclopenta[a]inden-8-yl]-1-methyl-ethylamine (WO 02/051844);
(S)-2-(6-cyclopropoxy-2,3-dihydro-1H-3a-aza-cyclopenta[a]inden-8-yl)-1-methyl-ethylamine (WO 02/051844),
(S)-2-[8-(2-amino-propyl)- 2,3-dihydro-1H-3a-aza-cyclopenta[a]inden-6-yloxy]-1-ethanol (WO 02/051844),
(S)-2-[6-(3-methoxy-propoxy) 2,3-dihydro-1H-3a-aza-cyclopenta[a]inden-8-yl]-1-methyl-ethylamine (WO 02/051844);
(R,S)-2-(2,3-dihydro-1H-3a,6-diaza-cyclopenta[a]inden-8-yl)-1-methyl-ethylamine (WO 01/66548),
(R,S)-2-(2,3-dihydro-1H-3a,4-diaza-cyclopenta[a]inden-8-yl)-1-methyl-ethylamine (WO 01/66548),
(S)-2-(2,3-dihydro-1H-3a,5-diaza-cyclopenta[a]inden-8-yl)-1-methyl-ethylamine (WO 01/66548),
(R)-2-(2,3-dihydro-1H-3a,5-diaza-cyclopenta[a]inden-8-yl)-1-methyl-ethylamine (WO 01/66548),
(S)-2-(2,3-dihydro-1H-3a,6-diaza-cyclopenta[a]inden-8-yl)-1-methyl-ethylamine (WO 01/66548),
(R)-2-(2,3-dihydro-1H-3a,6-diaza-cyclopenta[a]inden-8-yl)-1-methyl-ethylamine (WO 01/66548),
2-(2,3-dihydro-1H-3a,6-diaza-cyclopenta[a]inden-8-yl)-1-methyl-ethylamine (WO 01/66548);
1-(5-chloroindazol-3-yl)-2-propylamine (WO 00/12482),
1-(5-chloro-1-methylindazol-3-yl)-2-propylamine (WO 00/12482),
1-(5-chloro-1-isopropylindazol-3-yl)-2-propylamine (WO 00/12482),
1-(5-methoxyindazol-3-yl)-2-propylamine (WO 00/12482),
1-(5-methoxy-1-methylindazol-3-yl)-2-propylamine (WO 00/12482),
1-(5-bromoindazol-3-yl)-2-propylamine (WO 00/12482);
[8-(2,4-dichlorophenyl)-2,3,5-tetrahydro[1,4]oxazino-[2,3,4-hi]indol-6-yl]methanamine (WO 03/024976);
1-(1H-pyrrolo[2,3-f]quinolin-1-yl-2-propylamine (WO 00/12502),
1-(1H-pyrrolo[3,2-h]isoquinolin-1-yl)-2-propylamine (WO 00/12502),
1-(5-chloro-1H-pyrrolo[2,3-f]quinolin-1-yl)-2-propylamine (WO 00/12502),
1-(1H-pyrrolo[2,3-f]isoquinolin-1-yl)-2-propylamine (WO 00/12502);
(S)-1-(7,8-difluoro-1,2,3,4-tetrahydrocyclopent[b]indol-4-yl)-2-propylamine (WO 01/12603),
(S)-1-(7-fluoro-1,2,3,4-tetrahydrocyclopent[b]indol-4-yl)-2-propylamine (WO 01/12603),
(S)-1-(8-chloro-1,2,3,4-tetrahydrocyclopent[b]indol-4-yl)-2-propylamine (WO 01/12603),
(S)-1-(6-methoxy-1,2,3,4-tetrahydrocyclopent[b]indol-4-yl)-2-propylamine (WO 01/12603),
(S)-1-(7-fluoro-6-methoxy-1,2,3,4-tetrahydrocyclopent[b]indol-4-yl)-2-propylamine (WO 01/12603),
(S)-1-(7-fluoro-8-methoxy-1,2,3,4-tetrahydrocyclopent[b]indol-4-yl)-2-propylamine (WO 01/12603),
(S)-1-(8-chloro-7-fluoro-1,2,3,4-tetrahydrocyclopent[b]indol-4-yl)-2-propylamine (WO 01/12603),
(S)-1-(1,2,3,4-tetrahydrocyclopent[b]indol-4-yl)-2-propylamine (WO 01/12603),
(R)-1-(1,2,3,4-tetrahydrocyclopent[b]indol-4-yl)-2-propylamine (WO 01/12603);
1-(6-methylthioindazol-1-yl)-2-propylamine (WO 00/17170),
1-(6-phenylthioindazol-1-yl)-2-propylamine (WO 00/17170),
1-(6-methoxy-3-methylindazol-1-yl)-2-propylamine (WO 00/12481),
1-(5,6-difluoro-3-methylindazol-1-yl)-2-propylamine (WO 00/12481),
1-(6-chloro-5-fluoro-3-methylindazol-1-yl)-2-propylamine (WO 00/12481),
1-(3-ethyl-6-trifluoromethylindazol-1-yl)-2-propylamine (WO 00/12481),
1-(6-bromo-3-ethylindazol-1-yl)-2-propylamine (WO 00/12481),
1-(3-ethyl-6-methylthioindazol-1-yl)-2-propylamine (WO 00/12481);
2-(l H-furo[2,3-g]indazol-1-yl)ethylamine (WO 98/56768),
2-(7-bromo-1H-thieno[2,3-g]indazol-1-yl)ethylamine (WO 98/56768),
2-(7-iodo-1H-thieno[2,3-g]indazol-1-yl)ethylamine (WO 98/56768),
2-(7-methoxy-1H-thieno[2,3-g]indazol-1-yl)ethylamine (WO 98/56768),
(S)-2-(1H-furo[2,3-g]indazol-1-yl)-1-methylethylamine (WO 98/56768),
2-(7-methyl-1H-thieno[2,3-g]indazol-1-yl)ethylamine (WO 98/56768),
(S)-2-(7-methoxy-1H-thieno[2,3-g]indazol-1-yl)-1-methylethylamine (WO 98/56768),
(S)-1-methyl-2-(7-methyl-1H-thieno[2,3-g]indazol-1-yl)ethylamine (WO 98/56768),
2-(7-ethyl-1H-thieno[2,3-g]indazol-1-yl)ethylamine (WO 98/56768),
(S)-2-(7-ethyl-1H-thieno[2,3-g]indazol-1-yl)-1-methylethylamine (WO 98/56768);
1-(6-chloro-5-fluoroindolin-1-yl)-2-propylamine (WO 00/12475),
1-(5,6-difluoroindolin-1-yl)-2-propylamine (WO 00/12475),
1-(6-bromo-5-fluoroindolin-1-yl)-2-propylamine (WO 00/12475),
1-(6-bromoindolin-1-yl)-2-propylamine (WO 00/12475),
1-(6-chloroindolin-1-yl)-2-propylamine (WO 00/12475),
1-(5-fluoro-6-trifluoromethylindolin-1-yl)-2-propylamine (WO 00/12475),
1-(5-fluoro-6-methylthioindolin-1-yl)-2-propylamine (WO 00/12475),
1-(5-fluoro-6-iodoindolin-1-yl)-2-propylamine (WO 00/12475),
1-(5-fluoro-6-ethylthioindolin-1-yl)-2-propylamine (WO 00/12475),
1-(-5-fluoro-6-methylindolin-1-yl)-2-propylamine (WO 00/12475),
1-(6-methylthioindolin-1-yl)-2-propylamine (WO 00/12475),
1-(6-ethylthioindolin-1-yl)-2-propylamine (WO 00/12475),
1-(6-trifluoromethylindolin-1-yl)-2-propylamine (WO 00/12475),
1-(6-methoxyindolin-1-yl)-2-propylamine (WO 00/12475),
1-(6-propylthioindolin-1-yl)-2-propylamine (WO 00/12475),
1-(6-isopropylthioindolin-1-yl)-2-propylamine (WO 00/12475),
2-(6-chloroindolin-1-yl)-1-ethylamine (WO 00/12475),
2-(6-bromoindolin-1-yl)-1-ethylamine (WO 00/12475),
1-(5-chloroindolin-1-yl)-2-propylamine (WO 00/12475),
1-(5-fluoroindolin-1yl)-2-propylamine (WO 00/12475),
1-(6-methylindolin-1-yl)-2-propylamine (WO 00/12475),
(S)-1-[1-(1,2,3,6,7,8-hexahydrocyclopent[g]indolyl)]-2-propylamine (WO 01/12602),
(S)-1-(2,3,7,8-tetrahydrofuro[2,3-g]indol-1-yl)-2-propylamine (WO 01/12602),
(R)-6-thienyl-4-methyl-1,2,3,4-tetrahydro-pyrazino [1,2-a] indole (WO 02/072584),
(R)-4,6-dimethyl-1,2,3,4-tetrahydro-pyrazino[1,2-a]indole (WO02/072584),
(R)-7-chloro-4-methyl-1,2,3,4-tetrahydro-pyrazino[1,2-a] indole (WO 02/072584),
(R)-4-methyl-6-trifluoromethyl-1,2,3,4-tetrahydro-pyrazino[1,2-a] indole (WO 02/072584),
(R)-6-ethyl-8-fluoro-4-methyl-1,2,3,4-tetrahydro-pyrazino [1,2-a] indole (WO 02/072584),
(R)-8-fluoro-4,7-dimethyl-1,2,3,4-tetrahydro-pyrazino[1,2-a] indole (WO 02/072584),
(R)-6-fluoro-4,7-dimethyl-1,2,3,4-tetrahydro-pyrazino [1,2-a] indole (WO 02/072584),
(R)-4-methyl-1,2,3,4-tetrahydro-pyrazino[1,2-a]indole-6-carbonitrile (WO 02/072584),
(R)-4,6,10-trimethyl-1,2,3,4-tetrahydro-pyrazino[1,2-a]indole (WO 02/072584),
(R)-8,9-dichloro-2,3,4,4a-tetrahydro-1H-pyrazino[1,2-a]quinoxalin-5(6H)-one (WO 00/35922),
8,9-dichloro-2,3,4,4a,5,6-hexahydro-1H-pyrazino[1,2-a]quinoxaline (WO 00/35922),
8-(2,4-dichlorophenyl)-2,3,4,4a,5,9b-hexahydro-1H-pyrido[4,3-b]-indole (WO 03/014118),
6-bromo-8-(2,4-dichlorophenyl)-2,3,4,4a,5,9b-hexahydro-1H-pyrido[4,3-b]-indole (WO 03/014118)
2-phenyl-5,6,6b,7,8,9,10,10a-octahydro-4-oxa-3,6a,9-triazafluoranthene (WO 03/033497),
2-(2,6-difluorophenyl)-5,6,6b,7,8,9,10,10a-octahydro-4-oxa-3,6a,9-triaza-fluoranthene (WO 03/033497),
2-(2,4-dichlorophenyl)-5,6,6b,7,8,9,10,10a-octahydro-4-oxa-3,6a,9-triaza-fluoranthene (WO 03/033497),
2-phenyl-6,7,7b,8,9,10,11,11a-octahydro-5H-4-thia-3,7a,10-triazacyclohepta[jk]fluorene (WO 03/033497),
2-(2,4-dichlorophenyl)-6,7,7b,8,9,10,11,11a-octahydro-5H-4-thia-3,7a,10-triazacyclohepta[jk]fluorene (WO 03/033497),
2-(2,6-difluorophenyl)-6,7,7b,8,9,10,11,11a-octahydro-5H-4-thia-3,7a,10-triazacyclohepta[jk]fluorene (WO 03/033497);
5-(2,4-dichlorophenyl)-10a-methyl-1,2,6b,7,8,9,10,10a-octahydro[1,4]oxazino[2,3,4-hi]pyrido[4,3-b]indole (WO 03/014125),
5-(2,6-difluorophenyl)-10a-methyl-1,2,6b,7,8,9,10,10a-octahydro[1,4]oxazino[2,3,4-hi]pyrido[4,3-b]indole (WO 03/014125),
5-(2,4-dichlorophenyl)-10a-ethyl-1,2,6b,7,8,9,10,10a-octahydro[1,4]oxazino[2,3,4-hi]pyrido[4,3-b]indole (WO 03/014125),
5-(2,6-difluorophenyl)-10a-ethyl-1,2,6b,7,8,9,10,10a-octahydro[1,4]oxazino[2,3,4-hi]pyrido[4,3-b]indole (WO 03/014125),
5-(2,4-dichlorophenyl)-10a-methyl-1,2,6b,7,8,9,10,10a-octahydropyrido[4,3-b][1,4]thiazino[2,3,4-hi]indole (WO 03/014125),
5-(2,6-difluorophenyl)-10a-methyl-1,2,6b,7,8,9,10,10a-octahydropyrido[4,3-b][1,4]thiazino[2,3,4-hi]indole (WO 03/014125),
5-(2,4-dichlorophenyl)-10a-ethyl-1,2,6b,7,8,9,10,10a-octahydropyrido[4,3-b][1,4]thiazino[2,3,4-hi]indole (WO 03/014125),
5-(2,6-difluorophenyl)-10a-ethyl-1,2,6b,7,8,9,10,10a-octahydropyrido[4,3-b][1,4]thiazino[2,3,4-hi]indole (WO 03/014125),
tert-butyl-6b-methyl-1,2,6b,9,10,10a-hexahydro[1,4]oxazino[2,3,4-hi]pyrido[4,3-b]indole-8(7H)-carboxylate (WO 03/014125),
S-4-[(2-propylamino)carbonyl]oxybenzylpiperazine-1-thiocarboxylate (WO 0248124),
S-4-[(benzylamino)carbonyl]oxybenzylpiperazine-1-thiocarboxylate (WO 0248124),
S-4-[(tert-butylamino)carbonyl]oxybenzylpiperazine-1-thiocarboxylate (WO 0248124),
2,6-difluoro-4-difluoromethoxybenzyl-cis-2,6-dimethylpiperazine-1 carboxylate (WO 0248124),
(R)-4-difluoromethoxybenzyl-2-ethylpiperazine-1-carboxylate (WO 0248124),
(R)-2,6-difluoro-4-propoxybenzyl-2-methylpiperazine-1-carboxylate (WO 0248124),
cis-2,6-dimethyl-piperazine-1-carboxylic acid 4- (3,5-dimethyl-isoxazol-4 ylmethoxy)-2,6-difluoro-benzyl ester (WO 0248124),
2-fluoro-5-(2-propenyl)oxybenzyl cis-2,6-dimethylpiperazine-1-carboxylate (WO 0248124),
(R)-2-fluoro-5-pentyloxybenzyl-2-methylpiperazine-1-carboxylate (WO 0248124),
5-(cyclopropylmethyl)oxy-2-fluorobenzyl-cis-2,6-dimethylpiperazine-1-carboxylate (WO 0248124),
(R)-2-ethyl-piperazine-1-carboxylic acid-4-cyclopropylmethoxy-2,6-difluoro-benzyl ester (WO 0248124),
(R)-2-ethyl-piperazine-1-carboxylic acid-2,6-difluoro-4-propoxy-benzyl ester (WO 0248124),
(R)-2-ethyl-piperazine-1-carboxylic acid-4-allyloxy-2,6-difluoro-benzyl ester (WO 0248124),
(R)-2-ethyl-piperazine-1-carboxylic acid-2,6-difluoro-4-prop-2-ynyloxy-benzyl ester (WO 0248124),
(R)-2-ethyl-piperazine-1-carboxylic acid-4-cyclopropylmethoxy-2-chloro-6-fluorobenzyl ester (WO 0248124),
(R)-2-ethyl-piperazine-1-carboxylic acid-2-chloro-6-fluoro-4-propoxy-benzyl ester (WO 0248124),
(R)-2-ethyl-piperazine-1-carboxylic acid-4-allyloxy-2-chloro-6-fluoro-benzyl ester (WO 0248124),
(R)-2-ethyl-piperazine-1-carboxylic acid-2-chloro-6-fluoro-4-prop-2-ynyloxy-benzyl ester (WO 0248124),
N,N-Dimethyl (2-(3-[2-(2-(R)-methyl-3,4,5,6-tetrahydro-2H-[1,2']bipyrazinyl-3'-yloxy)-ethoxy]-pyridin-2-yloxy)-ethyl-amine (WO 04/000830),
N,N-Diisopropyl-(2-(3-[2-(2-(R)-methyl-3,4,5,6-tetrahydro-2H-[1,2']bipyrazinyl-3'-yloxy)-ethoxy]-pyridin-2-yloxy)-ethyl-amine (WO 04/000830),
N,N-Dimethyl-2-[(3-{2-[(3-piperazin-1-ylpyrazin-2-yl)oxy]ethoxy}pyridin-2-yl)oxyethanamine (WO 04/000830),
2-[(2R)-2-Methylpiperazin-1-yl]-3-(2-{[2-(2-pyrrolidin-1-ylethoxy)pyridin-3-yl]oxy}ethoxy)pyrazine (WO 04/000830),
N,N-Dimethyl-4-({3-[2-{3-[(2R)-2-methylpiperazin-1-yl]pyrazin-2-yl}oxy)ethoxy]pyridin-2-yl}oxy)butan-1-amine (WO 04/000830),
N-Methyl-N-[2-({3-[2-({3-[(2R)-2-methylpiperazin-1-yl]pyrazin-2-yl}oxy)ethoxy]pyridin-2-yl}oxy)ethyl]propan-2-amine (WO 04/000830),
N,N-Dimethyl-3-({3-[2-({3-[(2R)-2-methylpiperazin-1-yl]pyrazin-2-yl}oxy)ethoxy]pyridin-2-yl}oxy)propan-1-amine (WO 04/000830),
N,N,2-Trimethyl-1-({3-[2-({3-[(2R)-2-methylpiperazin-1-yl]pyrazin-2-yl}oxy)ethoxy]pyridin-2-yl}oxy)propan-2-amine (WO 04/000830),
[2-({3-[2-({3-[(2R)-2-Methylpiperazin-1-yl]pyrazin-2-yl}oxy)ethoxy]pyridin-2-yl}oxy)ethyl]amine (WO 04/000830),
N-Methyl-2-({3-[2-({3-[(2R)-2-methylpiperazin-1-yl]pyrazin-2-yl}oxy)ethoxy]pyridin-2-yl}oxy)ethanamine (WO 04/000830),
2-{2-[{2-[2-(Dimethylamino)ethoxy]pyridin-3-yl}oxy]ethoxy}-3-[(2R)-2,4-dimethylpiperazin-1-yl]pyrazine (WO 04/000830),
2-[2-(2-[2-(Dimethylamino)ethoxy]phenoxy)ethoxy]-3-[(2R)-2-methylpiperazin-1-yl]pyrazine (WO 04/000830),
{2-[2({3-[2-({3-[(2R)-2-Methylpiperazin-1-yl]pyrazin-2-yl}oxy)ethoxy]pyridin-2-yl}oxy)ethoxy]ethyl}amine (WO 04/000830),
[6-({3-[2-({3-[(2R)-2-Methylpiperazin-1-yl]pyrazin-2-yl}oxy)etyoxy]pyridin-2-yl}oxy)hexyl]amine (WO 04/000830),
[5-({3-[2-({3-[(2R)-2-Methylpiperazin-1-yl]pyrazin-2-yl}oxy)ethoxy]pyridin-2-yl}oxy)pentyl]amine (WO 04/000830),
5-({3-[2-({3-[(2R)-2,4-Dimethylpiperazin-1-yl]pyrazin-2-yl}oxy)ethoxy]pyridin-2-yl}oxy)-N,N-dimethylpentan-1-amine (WO 04/000830),
2-[(2R)-2-Methylpiperazin-1-yl]-3-(2-{[2-(2-piperazin-1-ylethoxy)pyridin-3-yl]oxy}ethoxy)pyrazine (WO 04/000830),
2-{2-[(2-{[2-(Dimethylamino)ethoxy]methyl}pyridin-3-yl)oxy]ethoxy}-3-[(2R)-2-methylpiperazin-1-yl]pyrazine (WO 04/000830),
2-{2-[{2-[3-(Dimethylamino)propyl]pyridin-3-yl}oxy]ethoxy}-3-[(2R)-2-methylpiperazin-1-yl]pyrazine (WO 04/000830),
2-{2-[{2-[(1Z)-3-(Dimethylamino)prop-1-enyl]pyridin-3-yl}oxy]ethoxy}-3-[(2R)-2-methylpiperazin-1-yl]pyrazine (WO 04/000830),
2-{2-[{2-[(1E)-3-(Dimethylamino)prop-1-enyl]pyridin-3-yl}oxy]ethoxy}-3-[(2R)-2-methylpiperazin-1-yl]pyrazine (WO 04/000830),
2-[(2R)-2-Methylpiperazin-1-yl]-3-[2-(2{2-[(1-methylpiperidin-4-yl)oxy]pyridin-3-yl}oxy)ethoxy]pyrazine, trifluoroacetate (WO 04/000830),
2-[(2R)-2-Methylpiperazin-1-yl]-3-[2-(2{2-[2-(1-methylpyrrolidin-2-yl)ethoxy]pyridin-3-yl}oxy)ethoxy]pyrazine, trifluoroacetate (WO 04/000830),
2-[(2R)-2-Methylpiperazin-1-yl]-3-[2-{[2-(piperidin-3-ylmethoxy)pyridin-3-yl]oxy}ethoxy)pyrazine, trifluoroacetate (WO 04/000830),
2-[(2R)-2-Methylpiperazin-1-yl]-3-{2-[(2-{[(2S)-1-methylpyrrolidin-2-yl]methoxy}pyridin-3-yl)oxy]ethoxy}pyrazine, trifluoroacetate (WO 04/000830);
4-(Benzyloxy)-2-(1-piperazinyl)pyrimidine (US 2004/014767),
4-[(2-Methoxybenzyl)oxy]-2-(1-piperazinyl)pyrimidine (US 2004/014767), or
2-{[3-(Benzyloxy)benzyl]oxy}-4-(1-piperazinyl)pyrimidine (US 2004/014767).

Preferred suitable 5-HT2C receptor agonists for use in the invention also include compounds included in patent application WO 03/000663 (preferably the compounds exemplified in WO 03/000663). These include compounds of Formula (IA) 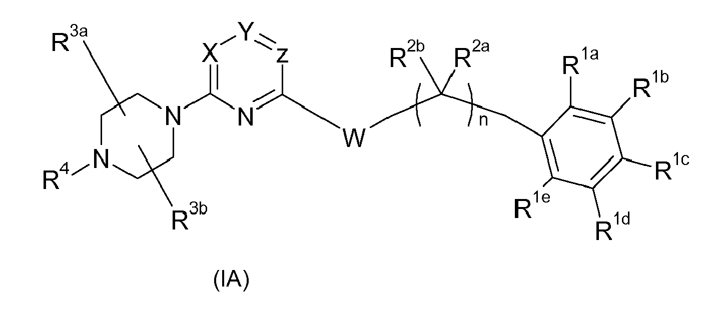 wherein
X and Y are CR and Z is N, or X is N and Y and Z are CR, where R for each occurrence is hydrogen, halogen, (C₁-C₄)alkyl, amino, or (C₁-C₄)alkylamino;
W is oxy, thio, amino, (C₁-C₄)alkylamino, or acetylamino;
at least one of R^{1a}, R^{1b}, R^{1d} and R^{1e} is independently selected from the group consisting of halogen, nitro, amino, cyano, -C(O)NH₂, (C₁-C₄)alkyl, halo-substituted (C₁-C₄)alkyl, (C₁-C₄) alkoxy, and halo-substituted(C₁-C₄)alkoxy, or R^{1a} and R^{1b} taken together form a five- or six-membered, aromatic or partially or fully saturated fused ring, or R^{1a} taken together with R^{2a} or R^{2b} forms a five-or six-membered, fully saturated fused ring;
R^{1c} is hydrogen;
R^{2a} and R^{2b} are each independently hydrogen, (C₁-C₄)alkyl, partially or fully saturated (C₃-C₆)cycloalkyl, or one of which taken together with R^{1a} forms a five- or six-membered, fully saturated fused ring;
n is 0, 1, or 2;
R^{3a} and R^{3b} are each independently hydrogen, (C₁-C₄)alkyl, or (C₁-C₄)alkyl substituted with hydroxy, fluoro, or (C₁-C₄)alkoxy;
R⁴ is hydrogen, hydroxy, (C₁-C₄)alkyl, (C₁-C₄)alkyl substituted with hydroxy or cyano, (C₁-C₄)alkylcarbonyl, (C₁-C₄)alkoxy, (C₁-C₄)alkoxy-carbonyl, (C₃-C₄)alkenyl, or an amino-protecting group;
Preferred compounds of Formula (IA) are those where X and Y are CR and Z is N, or X is N and Y and Z are CR, where R is hydrogen, chloro, fluoro, or methyl;
   (i) R^{1a} is halogen, (C₁-C₄)alkyl, trifluoromethyl, methoxy, or trifluoromethoxy, and R^{1b}, R^{1d} and R^{1e} are each hydrogen,
   (ii) R^{1b} is halogen, methyl, or methoxy and R^{1a}, R^{1d} and R^{1e} are each hydrogen,
   (iii) R^{1a} and R^{1b} are each independently halogen or methyl and R^{1d} and R^{1e} are each hydrogen,
   (iv) R^{1b} and R^{1d} are each independently halogen or methyl and R^{1a} and R^{1e} are each hydrogen,
   (v) R^{1a} and R^{1d} are each independently halogen or methyl and R^{1b} and R^{1e} are each hydrogen,
   (vi) R^{1a} and R^{1e} are each independently halogen or methyl and R^{1b} and R^{1d} are each hydrogen, or
   (vii) R^{1a}, R^{1b} and R^{1d} are each independently halogen or methyl and R^{1e} is hydrogen;
W is oxy or amino;
n is 1;
R^{2a} and R^{2b} are each independently methyl or hydrogen;
R^{3a} and R^{3b} are each independently hydrogen or (C₁-C₂)alkyl (preferably (2R)-methyl or (2R)-ethyl); and
R⁴ is hydrogen or (C₁-C₄)alkyl.

When Z is N, then X is preferably CH; Y is CR, where R for each occurrence is hydrogen or methyl; (i) R^{1a} is halogen, (C₁-C₄)alkyl, trifluoromethyl, methoxy, or trifluoromethoxy, and R^{1b}, R^{1d} and R^{1e} are each hydrogen, (ii) R^{1b} is halogen, methyl, or methoxy and R^{1a}, R^{1d} and R^{1e} are each hydrogen, (iii) R^{1a} and R^{1b} are each independently halogen or methyl and R^{1d} and R^{1e} are each hydrogen, (iv) R^{1b} and R^{1d} are each independently halogen or methyl and R^{1a} and R^{1e} are each hydrogen, (v) R^{1a} and R^{1d} are each independently halogen or methyl and R^{1b} and R^{1e} are each hydrogen, (vi) R^{1a} and R^{1e} are each independently halogen or methyl and R^{1b} and R^{1d} are each hydrogen, or (vii) R^{1a}, R^{1b} and R^{1d} are each independently halogen or methyl and R^{1e} is hydrogen; W is oxy or amino; n is 1; R^{2a} and R^{2b} are each independently methyl or hydrogen; and R^{3a} is hydrogen, (2R)-methyl, or (2R)-ethyl; and R^{3b} is hydrogen; and R⁴ is hydrogen or (C₁-C₄)alkyl.

When X is N, then Y is preferably CR, where R is hydrogen or methyl; Z is CH; (i) R^{1a} is halogen, (C₁-C₄)alkyl, trifluoromethyl, methoxy or trifluoromethoxy and R^{1b}, R^{1d} and R^{1e} are each hydrogen; (ii) R^{1b} is halogen, methyl, or methoxy and R^{1a}, R^{1d} and R^{1e} are each hydrogen, (iii) R^{1b} and R^{1d} are each independently halogen or methyl and R^{1a} and R^{1e} are each hydrogen, or (iv) R^{1a} and R^{1d} are each independently halogen or methyl and R^{1b} and R^{1e} are each hydrogen; W is amino; n is 1; R^{2a} and R^{2b} are each independently methyl or hydrogen; R^{3a} is hydrogen, (2R)-methyl, or (2R)-ethyl; and R^{3b} is hydrogen; and R⁴ is hydrogen or (C₁-C₄)alkyl.

Non-limiting examples of preferred compounds of Formula (IA) include;
2-(2-chloro-benzyloxy)-4-piperazin-1-yl-pyrimidine,
2-(3-fluoro-benzyloxy)-4-piperazin-1-yl-pyrimidine,
2-(3-chloro-benzyloxy)-4-piperazin-1-yl-pyrimidine,
2-(3-chloro-benzyloxy)-4-(2(R)-methyl-piperazin-1-yl)-pyrimidine,
2-(3-methoxy-benzyloxy)-4-piperazin-1-yl-pyrimidine,
(3-chloro-benzyl)-(2-piperazin-1-yl-pyrimidin-4-yl)-amine,
(3-chloro-benzyl)-(4-piperazin-1-yl-pyrimidin-2-yl)-amine,
(3-fluoro-benzyl)-(4-piperazin-1-yl-pyrimidin-2-yl)-amine,
(3-fluoro-benzyl)-(2-piperazin-1-yl-pyrimidin-4-yl)-amine,
2-[1-(3-fluoro-phenyl)-ethoxy]-4-methyl-6-piperazin-1-yl-pyrimidine,
2-[1-(3-fluoro-phenyl)-ethoxy]-4-piperazin-1-yl-pyrimidine,
2-[1-(2-chloro-phenyl)-ethoxy]-4-piperazin-1-yl-pyrimidine,
2-[1-(3-chloro-phenyl)-ethoxy]-4-piperazin-1-yl-pyrimidine,
2-[1-(3-chloro-phenyl)-ethoxy]-4-methyl-6-piperazin-1-yl-pyrimidine,
2-(2,3-difluoro-benzyloxy)-4-piperazin-1-yl-pyrimidine,
2-(2,5-difluoro-benzyloxy)-4-piperazin-1-yl-pyrimidine,
2-(2,5-difluoro-benzyloxy)-4-(2(R)-methyl-piperazin-1-yl)-pyrimidine,
2-(2,5-dichloro-benzyloxy)-4-piperazin-1-yl-pyrimidine,
2-(2,5-dichloro-benzyloxy)-4-(2(R)-methyl-piperazin-1-yl)-pyrimidine,
2-(3,5-dichloro-benzyloxy)-4-piperazin-1-yl-pyrimidine,
2-(3,5-difluoro-benzyloxy)-4-piperazin-1-yl-pyrimidine, or
4-piperazin-1-yl-2-(2,3,5-trifluoro-benzyloxy)-pyrimidine.

Preferred salts include 2-(2-chloro-benzyloxy)-4-piperazin-1-yl-pyrimidine, hydrochloride; 2-(3-fluoro-benzyloxy)-4-piperazin-1-yl-pyrimidine, hydrochloride; 2-(3-chloro-benzyloxy)-4-piperazin-1-yl-pyrimidine, hydrochloride; 2-(3-chloro-benzyloxy)-4-(2(R)-methyl-piperazin-1-yl)-pyrimidine, hydrochloride; (3-chloro-benzyl)-(2-piperazin-1-yl-pyrimidin-4-yl)-amine, hydrochloride; (3-chloro-benzyl)-(4-piperazin-1-yl-pyrimidin-2-yl)-amine, hydrochloride; (3-fluoro-benzyl)-(4-piperazin-1-yl-pyrimidin-2-yl)-amine, hydrochloride; (3-fluoro-benzyl)-(2-piperazin-1-yl-pyrimidin-4-yl)-amine, fumarate; 2-(2,3-difluoro-benzyloxy)-4-piperazin-1-yl-pyrimidine, hydrochloride; 2-(2,5-difluoro-benzyloxy)-4-piperazin-1-yl-pyrimidine, hydrochloride; 2-(2,5-dichloro-benzyloxy)-4-piperazin-1-yl-pyrimidine, hydrochloride; 2-(3,5-dichloro-benzyloxy)-4-piperazin-1-yl-pyrimidine, hydrochloride; or 2-(3,5-difluoro-benzyloxy)-4-piperazin-1-yl-pyrirnidine, hydrochloride.

Non-limiting examples of more preferred compounds of Formula (IA) include;
2-(3-chloro-benzyloxy)-4-piperazin-1-yl-pyrimidine,
2-(3-chloro-benzyloxy)-4-(2(R)-methyl-piperazin-1-yl)-pyrimidine,
(3-chloro-benzyl)-(4-piperazin-1-yl-pyrimidin-2-yl)-amine,
(3-chloro-benzyl)-(2-piperazin-1-yl-pyrimidin-4-yl)-amine,
(3-fluoro-benzyl)-(4-piperazin-1-yl-pyrimidin-2-yl)-amine,
(3-fluoro-benzyl)-(2-piperazin-1-yl-pyrimidin-4-yl)-amine,
2-(2,3-difluoro-benzyloxy)-4-piperazin-1-yl-pyrimidine,
2-(2,5-difluoro-benzyloxy)-4-piperazin-1-yl-pyrimidine,
2-(2,5-difluoro-benzyloxy)-4-(2(R)-methyl-piperazin-1-yl)-pyrimidine,
2-(2,5-dichloro-benzyloxy)-4-(2(R)-methyl-piperazin-1-yl)-pyrimidine,
2-(3,5-dichloro-benzyloxy)-4-piperazin-1-yl-pyrimidine, or
4-piperazin-1-yl-2-(2,3,5-trifluoro-benzyloxy)-pyrimidine.

Yet further 5HT2C receptor agonists for use in the invention are compounds of Formula (IC) also provided in WO 03/000663: 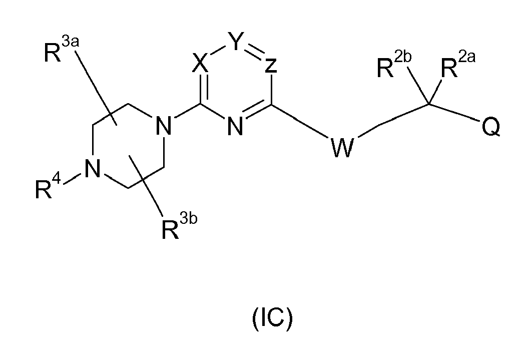 wherein
X and Y are CR and Z is N, or X is N and Y and Z are CR, where R for each occurrence is hydrogen, halogen, (C₁-C₄)alkyl, amino, or (C₁-C₄)alkylamino;
W is oxy, thio, amino, (C₁-C₄)alkylamino, or acetylamino;
Q is a heteroaryl group selected from the group consisting of pyridin-2-yl, pyridin-3-yl, furan-3-yl, furan-2-yl, thiophen-2-yl, thiophen-3-yl, thiazol-2-yl, pyrrol-2-yl, pyrrol-3-yl, pyrazol-3-yl, quinolin-2-yl, quinolin-3-yl, isoquinolin-3-yl, benzofuran-2-yl, benzofuran-3-yl, isobenzofuran-3-yl, benzothiophen-2-yl, benzothiophen-3-yl, indol-2-yl, indol-3-yl, 2H-imidazol-2-yl, oxazol-2-yl, isoxazol-3-yl, 1,2,4-oxadiazol-3-yl, 1,2,4-triazol-3-yl, and 1,2,4-oxathiazol-3-yl, where said heteroaryl group is optionally substituted with one to three substituents independently selected from halo, (C₁-C₄)alkyl or (C₁-C₄)alkyoxy;
R^{2a} and R^{2b} are each independently hydrogen, (C₁-C₄)alkyl, or partially or fully saturated (C₃-C₆)cycloalkyl;
R^{3a} and R^{3b} are each independently hydrogen, (C₁-C₄)alkyl, or (C₁-C₄)alkyl substituted with hydroxy, fluoro, or (C₁-C₄)alkoxy;
R⁴ is hydrogen, hydroxy, (C₁-C₄)alkyl, (C₁-C₄)alkyl substituted with hydroxy or cyano, (C₁-C₄)alkylcarbonyl, (C₁-C₄)alkoxy, (C₁-C₄)alkoxy-carbonyl, (C₃-C₄)alkenyl, or an amino-protecting group.

Non-limiting examples of preferred compounds of Formula (IC) include: 4-piperazin-1-yl-2-(pyridin-2-ylmethoxy)-pyrimidine, 2-(6-methyl-pyridin-2-ylmethoxy)-4-piperazin-1-yl-pyrimidine, or 2-(6-chloro-pyridin-2-ylmethoxy)-4-piperazin-1-yl-pyrimidine.

Non-limiting examples of more preferred compounds of Formula (IC) include 2-(6-methyl-pyridin-2-ylmethoxy)-4-piperazin-1-yl-pyrimidine or 2-(6-chloro-pyridin-2-ylmethoxy)-4-piperazin-1-yl-pyrimidine.

Some of the compounds described in WO 03/000663 contain at least one chiral center; consequently, those skilled in the art will appreciate that all stereoisomers (e.g., enantiomers and diasteroisomers) of these compounds may be used within the scope of the present invention. In addition, tautomeric forms of the compounds may also be used within the scope of the present invention.

Yet further suitable 5HT2C receptor agonists for use in the invention are compounds provided in WO 03/000663 of Formula (IB): 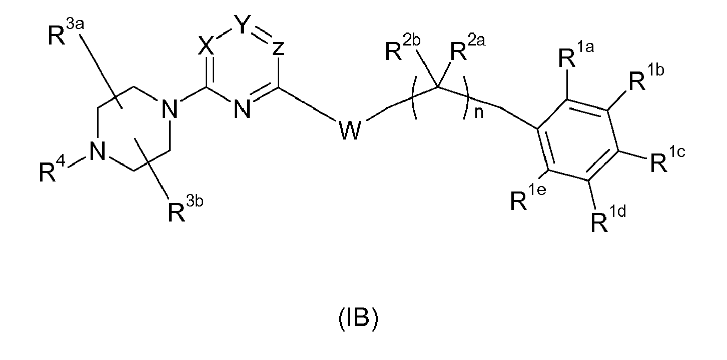 wherein
X and Y are CR and Z is N, or X is N and Y and Z are CR, where R for each occurrence is hydrogen, halogen, (C₁-C₄)alkyl, amino, or (C₁-C₄)alkylamino;
W is oxy, thio, amino, (C₁-C₄)alkylamino, or acetylamino;
R^{1a}, R^{1b}, R^{1c}, R^{1d} and R^{1e} are each independently hydrogen, halogen, nitro, cyano, amino, (C₁-C₄)alkyl, halo-substituted (C₁-C₄)alkyl, (C₁-C₄)alkoxy, halo-substituted (C₁-C₄)alkoxy, -C(O)NH₂, R^{1a} and R^{1b} taken together form a five- or six-membered, aromatic or partially or fully saturated fused ring, or R^{1a} taken together with R^{2a} or R^{2b} forms a five- or six-membered, fully saturated, fused ring;
R^{2a} and R^{2b} are each independently hydrogen, (C₁-C₄)alkyl, partially or fully saturated (C₃-C₆)cycloalkyl, or one of which taken together with R^{1a} forms a five- or six-membered, fully saturated fused ring;
n is 0, 1, or 2;
R^{3a} and R^{3b} are each independently hydrogen, (C₁-C₄)alkyl, (C₁-C₄)alkyl substituted with hydroxy, fluoro, or (C₁-C₄)alkoxy;
R⁴ is hydrogen, hydroxy, (C₁-C₄)alkyl, (C₁-C₄)alkyl substituted with hydroxy or cyano, (C₁-C₄)alkylcarbonyl, (C₁-C₄)alkoxy, (C₁-C₄)alkoxy-carbonyl, or (C₃-C₄)alkenyl.

Non-limiting examples of preferred compounds of Formula (IB) include
2-benzyloxy-4-methyl-6-piperazin-1-yl-pyrimidine,
2-benzyloxy-4-piperazin-1-yl-pyrimidine,
4-benzyloxy-2-piperazin-1-yl-pyrimidine,
benzyl-(4-piperazin-1-yl-pyrimidin-2-yl)-amine,
benzyl-(2-piperazin-1-yl-pyrimidin-4-yl)-amine,
2-(3-fluoro-benzyloxy)-4-piperazin-1-yl-pyrimidine,
2-(3-chloro-benzyloxy)-4-piperazin-1-yl-pyrimidine,
2-(3-chloro-benzyloxy)-4-(2(R)-methyl-piperazin-1-yl)-pyrimidine,
2-(3-methoxy-benzyloxy)-4-piperazin-1-yl-pyrimidine,
2-(2-chloro-benzyloxy)-4-piperazin-1-yl-pyrimidine,
(3-chloro-benzyl)-(2-piperazin-1-yl-pyrimidin-4-yl)-amine,
(3-chloro-benzyl)-(4-piperazin-1-yl-pyrimidin-2-yl)-amine,
(3-fluoro-benzyl)-(4-piperazin-1-yl-pyrimidin-2-yl)-amine,
(3-fluoro-benzyl)-(2-piperazin-1-yl-pyrimidin-4-yl)-amine,
2-[1-(3-fluoro-phenyl)-ethoxy]-4-methyl-6-piperazin-1-yl-pyrimidine,
2-[1-(3-fluoro-phenyl)-ethoxyl-4-piperazin-1-yl-pyrimidine,
2-[1-(2-chloro-phenyl)-ethoxy]-4-piperazin-1-yl-pyrimidine,
2-[1-(3-chloro-phenyl)-ethoxy]-4-piperazin-1-yl-pyrimidine,
2-[1-(3-chloro-phenyl)-ethoxy]-4-methyl-6-piperazin-1-yl-pyrimidine,
2-(2,3-difluoro-benzyloxy)-4-piperazin-1-yl-pyrimidine,
2-(2,5-difluoro-benzyloxy)-4-piperazin-1-yl-pyrimidine,
2-(2,5-difluoro-benzyloxy)-4-(2(R)-methyl-piperazin-1-yl)-pyrimidine,
2-(2,5-dichloro-benzyloxy)-4-piperazin-1-yl-pyrimidine,
2-(2,5-dichloro-benzyloxy)-4-(2(R)-methyl-piperazin-1-yl)-pyrimidine,
2-(3,5-dichloro-benzyloxy)-4-piperazin-1-yl-pyrimidine,
2-(3,5-difluoro-benzyloxy)-4-piperazin-1-yl-pyrimidine, or
4-piperazin-1-yl-2-(2,3,5-trifluoro-benzyloxy)-pyrimidine.
Non-limiting examples of more preferred compounds include
2-benzyloxy-4-methyl-6-piperazin-1-yl-pyrimidine,
benzyl-(2-piperazin-1-yl-pyrimidin-4-yl)-amine,
benzyl-(4-piperazin-1-yl-pyrimidin-2-yl)-amine,
4-benzyloxy-2piperazin-1-yl-pyrimidine,
2-benzyloxy-4-piperazin-1-yl-pyrimidine,
2-(3-chloro-benzyloxy)-4-piperazin-1-yl-pyrimidine,
2-(3-chloro-benzyloxy)-4-(2(R)-methyl-piperazin-1-yl)-pyrimidine,
(3-chloro-benzyl)-(4-piperazin-1-yl-pyrimidin-2-yl)-amine,
(3-chloro-benzyl)-(2-piperazin-1-yl-pyrimidin-4-yl)-amine,
(3-fluoro-benzyl)-(4-piperazin-1-yl-pyrimidin-2-yl)-amine,
(3-fluoro-benzyl)-(2-piperazin-1-yl-pyrimidin-4-yl)-amine,
2-(2,3-difluoro-benzyloxy)-4-piperazin-1-yl-pyrimidine,
2-(2,5-difluoro-benzyloxy)-4-piperazin-1-yl-pyrimidine,
2-(2,5-difluoro-benzyloxy)-4-(2(R)-methyl-piperazin-1-yl)-pyrimidine,
2-(2,5-dichloro-benzyloxy)-4-(2(R)-methyl-piperazin-1-yl)-pyrimidine,
2-(3,5-dichloro-benzyloxy)-4-piperazin-1-yl-pyrimidine, or
4-piperazin-1-yl-2-(2,3,5-trifluoro-benzyloxy)-pyrimidine.

Suitable 5-HT2C receptor agonists for use in the invention also include compounds included in patent application WO 03/000666 (preferably the compounds exemplified in WO 03/000666).

These include compounds of Formula (IIA): 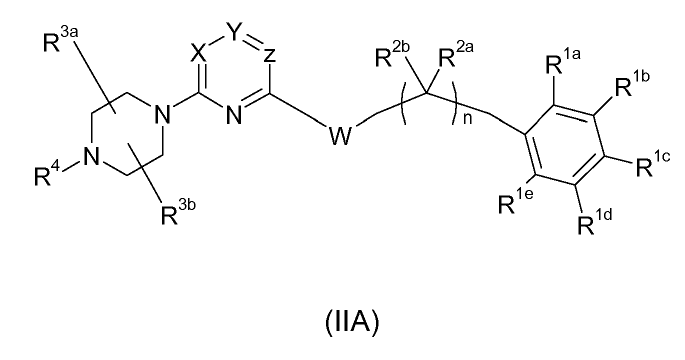 wherein
Y is nitrogen; X and Z are each independently CR, where R for each occurrence is hydrogen, halogen, (C₁-C₄)alkyl, amino, or (C₁-C₄)alkylamino;
W is oxy, thio, amino, (C₁-C₄)alkylamino, or acetylamino;
at least one of R^{1a}, R^{1b}, R^{1d}, and R^{1e} is independently selected from the group consisting of halogen, nitro, amino, (C₁-C₄)alkylamino, cyano, -C(O)NH₂, (C₁-C₄)alkyl, halo-substituted (C₁-C₄)alkyl, (C₁-C₄) alkoxy, and halo-substituted (C₁-C₄)alkoxy, or R^{1a} and R^{1b} taken together form a five- or six-membered, aromatic or partially or fully saturated fused ring, or R^{1a} taken together with R^{2a} or R^{2b} forms a five- or six-membered, fully saturated fused ring;
R^{1c} is hydrogen;
R^{2a} and R^{2b} are each independently hydrogen, (C₁-C₄)alkyl, partially or fully saturated (C₃-C₆)cycloalkyl, or one of which taken together with R^{1a} forms a five- or six-membered, fully saturated fused ring;
n is 0, 1, or 2;
R^{3a} and R^{3b} are each independently hydrogen, halogen, (C₁-C₄)alkyl, or (C₁-C₄)alkyl substituted with hydroxy, fluoro, or (C₁-C₄)alkoxy;
R⁴ is hydrogen, hydroxy, (C₁-C₄)alkyl, (C₁-C₄)alkyl substituted with hydroxy or cyano, (C₁-C₄)alkylcarbonyl, (C₁-C₄)alkoxy, (C₁-C₄)alkoxy-carbonyl, (C₃-C₄)alkenyl, or an amino-protecting group.

Preferred compounds of Formula (IIA) are those where Y is nitrogen; X and Z are each independently CR, where R is hydrogen, chloro, fluoro, or methyl;
i) R^{1a} is halogen, (C₁-C₄)alkyl, trifluoromethyl, methoxy, or trifluoromethoxy, and R^{1b}, R^{1d} and R^{1e} are each hydrogen,
(ii) R^{1b} is halogen, methyl, or methoxy and R^{1a}, R^{1d} and R^{1e} are each hydrogen,
(iii) R^{1a} and R^{1b} are each independently halogen or methyl and R^{1d} and R^{1e} are each hydrogen,
(iv) R^{1b} and R^{1d} are each independently halogen or methyl and R^{1a} and R^{1e} are each hydrogen,
(v) R^{1a} and R^{1d} are each independently halogen or methyl and R^{1b} and R^{1e} are each hydrogen,
(vi) R^{1a} and R^{1e} are each independently halogen or methyl and R^{1b} and R^{1d} are each hydrogen, or
(vii) R^{1a}, R^{1b} and R^{1d} are each independently halogen or methyl and R^{1e} is hydrogen;
W is oxy or amino; n is 1; R^{2a} and R^{2b} are each independently methyl or hydrogen; R^{3a} and R^{3b} are each independently hydrogen or (C₁-C₄)alkyl (preferably (2R)-methyl or (2R)-ethyl); and R⁴ is hydrogen or (C₁-C₄)alkyl.

More preferred compounds are those where Y is N; X and Z are each independently CR, where R is hydrogen or methyl; (i) R^{1a} is halogen, methyl, or trifluoromethyl and R^{1b}, R^{1d} and R^{1e} are each hydrogen, (ii) R^{1b} is halogen or methyl, and R^{1a}, R^{1d} and R^{1e} are each hydrogen, (iii) R^{1b} and R^{1d} are each independently halogen or methyl and R^{1a} and R^{1e} are each hydrogen, or (iv) R^{1a} and R^{1d} are each independently halogen or methyl and R^{1b} and R^{1e} are each hydrogen; W is oxy or amino; n is 1; R^{2a} and R^{2b} are each independently methyl or hydrogen; R^{3a} is hydrogen, (2R)-methyl, or (2R)-ethyl; R^{3b} is hydrogen; and R⁴ is hydrogen or (C₁-C₄)alkyl.

Most preferred are those compounds where Y is N, X and Z are each independently CR, where R for each occurrence is hydrogen or methyl; (i) R^{1a} is halogen, methyl, or trifluoromethyl and R^{1b}, R^{1d} and R^{1e} are each hydrogen, (ii) R^{1b} is halogen or methyl and R^{1a}, R^{1d} and R^{1e} are each hydrogen, (iii) R^{1b} and R^{1d} are each independently halogen or methyl and R^{1a} and R^{1e} are each hydrogen, or (iv) R^{1a} and R^{1d} are each independently halogen or methyl and R^{1b} and R^{1e} are each hydrogen; W is oxy or amino; n is 1; R^{2a} and R^{2b} are each independently methyl or hydrogen; R^{3a} is hydrogen, (2R)-methyl, or (2R)-ethyl; and R^{3b} is hydrogen; and R⁴ is hydrogen or (C₁-C₄)alkyl.

Non-limiting examples of preferred compounds of Formula (IIA) include: 6'-(3-chloro-benzyloxy)-3,4,5,6-tetrahydro-2H-[1,2']bipyrazinyl; 6'-(3-chloro-benzyloxy)-2-ethyl-3,4,5,6-tetrahydro-2H-[1,2']bipyrazinyl; 6'-(3-fluoro-benzyloxy)-3,4,5,6-tetrahydro-2H-[1,2']bipyrazinyl; 6'-(3-chloro-benzyloxy)-(2R)-methyl-3,4,5,6-tetrahydro-2H-[1,2']bipyrazinyl; 6'-(3-fluoro-benzyloxy)-(2R)-methyl-3,4,5,6-tetrahydro-2H-[1,2']bipyrazinyl; 6'-(3-chloro-benzyloxy)-3,4,5,6-tetrahydro-2H-[1,2']bipyrazinyl 4'-oxide; 6'-(2,5-dichloro-benzyloxy)-3,4,5,6-tetrahydro-2H-[1,2']bipyrazinyl; 6'-(2-fluoro-benzyloxy)-3,4,5,6-tetrahydro-2H-[1,2']bipyrazinyl; 6'-(3-nitro-benzyloxy)-3,4,5,6-tetrahydro-2H-[1,2']bipyrazinyl; 3-(3,4,5,6-tetrahydro-2H-[1,2']bipyrazinyl-6'-yloxymethyl)-b enzonitrile; 6'-(2,5-difluoro-benzyloxy)-3,4,5,6-tetrahydro-2H-[1,2']bipyrazinyl; 5'-bromo-6'-(3-chloro-benzyloxy)-3,4,5,6-tetrahydro-2H-[1,2']bipyrazinyl; 6'-(3-bromo-benzyloxy)-3,4,5,6-tetrahydro-2H-[1,2']bipyrazinyl; 3-(3,4,5,6-tetrahydro-2H-[1,2']bipyrazinyl-6'-yloxymethyl)-phenylamine; 6'-(2-methyl-benzyloxy)-3,4,5,6-tetrahydro-2H-[1,2']bipyrazinyl; 6'-(3-chloro-benzyloxy)-5'-fluoro-3,4,5,6-tetrahydro-2H-[1,2']bipyrazinyl; 5'-chloro-6'-(3-chloro-benzyloxy)-3,4,5,6-tetrahydro-2H-[1,2']bipyrazinyl; 6'-(indan-(1S)-yloxy)-3,4,5,6-tetrahydro-2H-[1,2']bipyrazinyl; 6'-(3-methy-benzyloxy)-3,4,5,6-tetrahydro-2H-[1,2']bipyrazinyl; 6'-(3-chloro-benzyloxy)-3,4,5,6-tetrahydro-2H-[1,2']bipyrazinyl-3'-ylamine; 6'-(2-chloro-benzyloxy)-3,4,5,6-tetrahydro-2H-[1,2']bipyrazinyl; 6'-[2-(3-chloro-phenyl)-ethoxy]-3,4,5,6-tetrahydro-2H-[1,2']bipyrazinyl; 6'-(2-chloro-benzyloxy)-(2R)-methyl-3,4,5,6-tetrahydro-2H-[1,2']bipyrazinyl; 6'-(3,4-difluoro-benzyloxy)-3,4,5,6-tetrahydro-2H-[1,2']bipyrazinyl; 6'-(3,5-difluoro-benzyloxy)-3,4,5,6-tetrahydro-2H-[1,2']bipyrazinyl; (3-fluoro-benzyl)-(3,4,5,6-tetrahydro-2H-[1,2']bipyrazinyl-6'-yl)-amie; (3-chloro-benzyl)-(3,4,5,6-tetrahydro-2H-[1,2']bipyrazinyl-6'-yl)-amine; (3,5-difluoro-benzyl)-(3,4,5,6-tetrahydro-2H-[1,2']bipyrazinyl-6'-yl)-amine; 3-[(3,4,5,6-tetrahydro-2H-[1,2']bipyrazinyl-6'-ylamino)-methyl]-b enzonitrile; (2,5-difluoro-benzyl)-(3,4,5,6-tetrahydro-2H-[1,2']bipyrazinyl-6'-yl)-amine; (3,5-dichloro-benzyl)-(3,4,5,6-tetrahydro-2H-[1,2']bipyrazinyl-6'-yl)-amine; (3-chloro-benzyl)-(2-methyl-3,4,5,6-tetrahydro-2H-[1,2']bipyrazinyl-6'-yl)-amine; (3-fluoro-benzyl)-(2-methyl-3,4,5,6-tetrahydro-2H-[1,2']bipyrazinyl-6'-yl)-amine; (2-chloro-benzyl)-(3,4,5,6-tetrahydro-2H-[1,2']bipyrazinyl-6'-yl)-amine; (2-chloro-6-fluoro-benzyl)-(3,4,5,6-tetrahydro-2H-[1,2']bipyrazinyl-6'-yl)-amine; (2,3-difluoro-benzyl)-(3,4,5,6-tetrahydro-2H-[1,2']bipyrazinyl-6'-yl)-amine; N-(3-chloro-benzyl)-N-(3,4,5,6-tetrahydro-2H-[1,2']bipyrazinyl-6'-yl)-acetamide; 6'-(3-chloro-benzylsulfanyl)-3,4,5,6-tetrahydro-2H-[1,2']bipyrazinyl; or 6'-benzylsulfanyl-3,4,5,6-tetrahydro-2H-[1,2']bipyrazinyl.

Non-limiting examples of more preferred compounds of Formula (IIA) include: 6'-(3-chloro-benzyloxy)-3,4,5,6-tetrahydro-2H-[1,2']bipyrazinyl; 6'-(3-chloro-benzyloxy)-(2R)-methyl-3,4,5,6-tetrahydro-2H-[1,2']bipyrazinyl; 6'-(2-chloro-benzyloxy)-(2R)-methyl-3,4,5,6-tetrahydro-2H-[1,2']bipyrazinyl; 6'-(3-fluoro-benzyloxy)-(2R)-methyl-3,4,5,6-tetrahydro-2H-[1,2']bipyrazinyl; 6'-(2-fluoro-benzyloxy)-3,4,5,6-tetrahydro-2H-[1,2']bipyrazinyl; 6'-(3-fluoro-benzyloxy)-3,4,5,6-tetrahydro-2H-[1,2']bipyrazinyl; 6'-(3-chloro-benzyloxy)-2-ethyl-3,4,5,6-tetrahydro-2H-[1,2']bipyrazinyl; 6'-(2,5-difiuoro-benzyloxy)-3,4,5,6-tetrahydro-2H-[1,2']bipyrazinyl; 6'-(2,5-dichloro-benzyloxy)-3,4,5,6-tetrahydro-2H-[1,2']bipyrazinyl; 6'-(3,5-difluoro-benzyloxy)-3,4,5,6-tetrahydro-2H-[1,2']bipyrazinyl; (3-fluoro-benzyl)-(3,4,5,6-tetrahydro-2H-[1,2']bipyrazinyl-6'-yl)-amine; (3-chloro-benzyl)-(3,4,5,6-tetrahydro-2H-[1,2']bipyrazinyl-6'-yl)-amine; (3,5-difluoro-benzyl)-(3,4,5,6-tetrahydro-2H-[1,2']bipyrazinyl-6'-yl)-amine; (3,5-dichloro-benzyl)-(3,4,5,6-tetrahydro-2H-[1,2']bipyrazinyl-6'-yl)-amine; (2-chloro-6-fluoro-benzyl)-(3,4,5,6-tetrahydro-2H-[1,2']bipyrazinyl-6'-yl)-amine; 6'-(3-chloro-benzyloxy)-3,4,5,6-tetrahydro-2H-[1,2']bipyrazinyl 4'-oxide; N-(3-chloro-benzyl)-N-(3,4,5,6-tetrahydro-2H-[1,2']bipyrazinyl-6'-yl)-acetamide; 6'-(3-chloro-benzylsulfanyl)-3,4,5,6-tetrahydro-2H-[1,2']bipyrazinyl; or 6'-(indan-(1S)-yloxy)-3,4,5,6-tetrahydro-2H-[1,2']bipyrazinyl.

Even more preferred compounds of the present invention include: 6'-(3-chloro-benzyloxy)-3,4,5,6-tetrahydro-2H-[1,2']bipyrazinyl; 6'-(3-fluoro-benzyloxy)-3,4,5,6-tetrahydro-2H-[1,2']bipyrazinyl; 6'-(3-chloro-benzyloxy)-3,4,5,6-tetrahydro-2H-[1,2']bipyrazinyl 4'-oxide; (3-fluoro-benzyl)-(3,4,5,6-tetrahydro-2H-[1,2']bipyrazinyl-6'-yl)-amine; 6'-(3-chloro-benzyloxy)-(2R)-methyl-3,4,5,6-tetrahydro-2H-[1,2']bipyrazinyl; 6'-(3,5-difluoro-benzyloxy)-3,4,5,6-tetrahydro-2H-[1,2']bipyrazinyl; 6'-(2,5-difluoro-benzyloxy)-3,4,5,6-tetrahydro-2H-[1,2']bipyrazinyl; or 6'-(indan-(1S)-yloxy)-3,4,5,6-tetrahydro-2H-[1,2']bipyrazinyl.

Preferred salts of the compounds described above include citrates, fumarates, hydrochlorides, L-malates, succinates, D,L-tartrates and more preferred salts include citrates, L-malates and D,L-tartrates.

Further suitable 5HT2C receptor agonists for use in the invention, provided in WO 03/000666, are compounds of Formula (IIC): 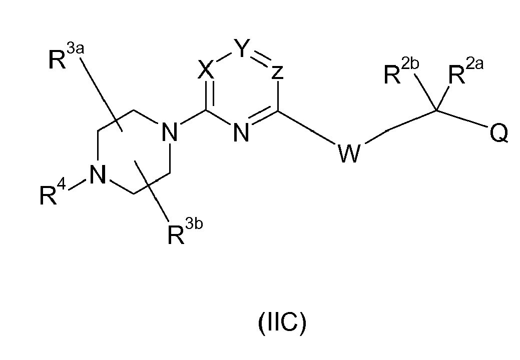 wherein
Y is N; X and Z are each independently CR, where R for each occurrence is hydrogen, halogen, (C₁-C₄)alkyl, amino, or (C₁-C₄)alkylamino;
W is oxy, thio, amino, (C₁-C₄)alkylamino, or acetylamino;
Q is a heteroaryl group selected from the group consisting of pyridin-2-yl, pyridin-3-yl, furan-3-yl, furan-2-yl, thiophen-2-yl, thiophen-3-yl, thiazol-2-yl, pyrrol-2-yl, pyrrol-3-yl, pyrazol-3-yl, quinolin-2-yl, quinolin-3-yl, isoquinolin-3-yl, benzofuran-2-yl, benzofuran-3-yl, isobenzofuran-3-yl, benzothiophen-2-yl, benzothiophen-3-yl, indol-2-yl, indol-3-yl, 2H-imidazol-2-yl, oxazol-2-yl, isoxazol-3-yl, 1,2,4-oxadiazol-3-yl, 1,2,4-oxadiazol-5-yl, 1,3,4-oxadiazol-2-yl, 1,3,4-oxadiazol-5-yl, 1,2,4-triazol-3-yl, 1,2,3-thiadiazol-4-yl, 1,2,3-thiadiazol-5-yl, 1,3,4-thiadiazol-2-yl, 1,3,4-thiadiazol-5-yl, and 1,2,4-oxathiazol-3-yl, where the heteroaryl group is optionally substituted with one to three substituents independently selected from halo, (C₁-C₄)alkyl, cyano, nitro, amino, (C₁-C₄)alkylamino, or (C₁-C₄)alkyoxy;
R^{2a} and R^{2b} are each independently hydrogen, (C₁-C₄)alkyl, or partially or fully saturated (C₃-C₆)cycloalkyl;
R^{3a} and R^{3b} are each independently hydrogen, halogen, (C₁-C₄)alkyl, or (C₁-C₄)alkyl substituted with hydroxy, fluoro, or (C₁-C₄)alkoxy;
R⁴ is hydrogen, hydroxy, (C₁-C₄)alkyl, (C₁-C₄)alkyl substituted with hydroxy or cyano, (C₁-C₄)alkylcarbonyl, (C₁-C₄)alkoxy, (C₁-C₄)alkoxy-carbonyl, or (C₃-C₄)alkenyl.

Non-limiting examples of preferred compounds of Formula (IIC) include: 6'-(pyridin-3-ylmethoxy)-3,4,5,6-tetrahydro-2H-[1,2']bipyrazinyl; 2-methyl-6'-(pyridin-3-ylmethoxy)-3,4,5,6-tetrahydro-2H-[1,2']bipyrazinyl; 6'-(thiophen-3-ylmethoxy)-3,4,5,6-tetrahydro-2H-[1,2']bipyrazinyl; 6'-([1,2,3]thiadiazol-4-ylmethoxy)-3,4,5,6-tetrahydro-2H-[1,2']bipyrazinyl; 6'-(6-fluoro-pyridin-2-ylmethoxy)-3,4,5,6-tetrahydro-2H-[1,2']bipyrazinyl; 2-methyl-6'-(6-methyl-pyridin-2-ylmethoxy)-3,4,5,6-tetrahydro-2H-[1,2']bipyrazinyl; 6'-(6-methyl-pyridin-2-ylmethoxy)-3,4,5,6-tetrahydro-2H-[1,2']bipyrazinyl; or 6'-(6-chloro-pyridin-2-ylmethoxy)-3,4,5,6-tetrahydro-2H-[1,2']bipyrazinyl.

Non-limiting examples of more preferred compounds of Formula (IIC) include 6'-(6-methyl-pyridin-2-ylmethoxy)-3,4,5,6-tetrahydro-2H-[1,2']bipyrazinyl; 6'-(6-chloro-pyridin-2-ylmethoxy)-3,4,5,6-tetrahydro-2H-[1,2']bipyrazinyl; 6'-(6-fluoro-pyridin-2-ylmethoxy)-3,4,5,6-tetrahydro-2H-[1,2']bipyrazinyl; 2-(6-chloro-pyridin-2-ylmethoxy)-4-piperazin-1-yl-pyrimidine; or 2-methyl-6'-(6-methyl-pyridin-2-ylmethoxy)-3,4,5,6-tetrahydro-2H-[1,2']bipyrazinyl.

Some of the compounds described above contain at least one chiral center; consequently, those skilled in the art will appreciate that all stereoisomers (e.g., enantiomers and diasteroisomers) of these compounds are within the scope of the present invention. In addition, tautomeric forms of the compounds are also within the scope of the present invention.

Further suitable 5HT2C receptor agonists for use in the invention provided in WO 03/000666 include a compound of Formula (IIB): 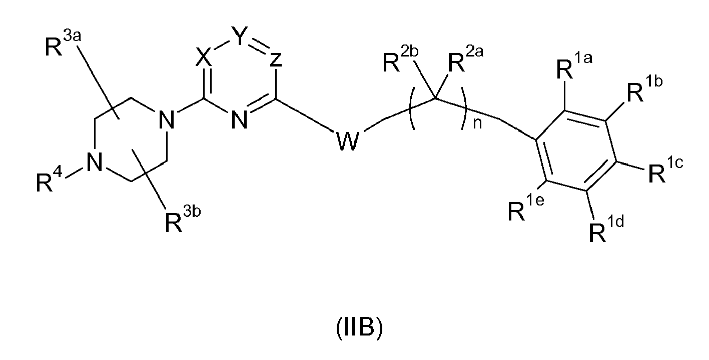 wherein
Y is N; X and Z are each independently CR, where R for each occurrence is hydrogen, halogen (preferably Cl or F), (C₁-C₄)alkyl, amino, or (C₁-C₄)alkylamino;
W is oxy, thio, amino, (C₁-C₄)alkylamino, or acetylamino;
R^{1a}, R^{1b}, R^{1c}, R^{1d} and R^{1e} are each independently hydrogen, halogen, nitro, cyano, amino, (C₁-C₄)alkylamino, (C₁-C₄)alkyl, halo-substituted (C₁-C₄)alkyl, (C₁-C₄)alkoxy, halo-substituted (C₁-C₄)alkoxy, -C(O)NH₂, R^{1a} and R^{1b} taken together form a five- or six-membered, aromatic or partially or fully saturated fused ring, or R^{1a} taken together with R^{2a} or R^{2b} forms a five- or six-membered, fully saturated, fused ring;
R^{2a} and R^{2b} are each independently hydrogen, (C₁-C₄)alkyl, partially or fully saturated (C₃-C₆)cycloalkyl, or one of which taken together with R^{1a} forms a five- or six-membered, fully saturated fused ring;
n is 0, 1, or 2;
R^{3a} and R^{3b} are each independently hydrogen, halogen, (C₁-C₄)alkyl, (C₁-C₄)alkyl substituted with hydroxy, fluoro, or (C₁-C₄)alkoxy;
R⁴ is hydrogen, hydroxy, (C₁-C₄)alkyl, (C₁-C₄)alkyl substituted with hydroxy or cyano, (C₁-C₄)alkylcarbonyl, (C₁-C₄)alkoxy, (C₁-C₄)alkoxy-carbonyl, or (C₃-C₄)alkenyl.

Non-limiting examples of preferred compounds of Formula (1 B) include 6'-benzyloxy-3,4,5,6-tetrahydro-2H-[1,2']bipyrazinyl; 6'-(3-chloro-benzyloxy)-3,4,5,6-tetrahydro-2H-[1,2']bipyrazinyl; 6'-(3-chloro-benzyloxy)-2-ethyl-3,4,5,6-tetrahydro-2H-[1,2']bipyrazinyl; 6'-(3-fluoro-benzyloxy)-3,4,5,6-tetrahydro-2H-[1,2']bipyrazinyl; 6'-(3-chloro-benzyloxy)-(2R)-methyl-3,4,5,6-tetrahydro-2H-[1,2']bipyrazinyl; 6'-(3-fluoro-benzyloxy)-(2R)-methyl-3,4,5,6-tetrahydro-2H-[1,2']bipyrazinyl; 6'-(3-chloro-benzyloxy)-3,4,5,6-tetrahydro-2H-[1,2']bipyrazinyl 4'-oxide; 6'-(2,5-dichloro-benzyloxy)-3,4,5,6-tetrahydro-2H-[1,2']bipyrazinyl; 6'-(2-fluoro-benzyloxy)-3,4,5,6-tetrahydro-2H-[1 ,2']bipyrazinyl; 6'-(3-nitro-benzyloxy)-3,4,5,6-tetrahydro-2H-[1,2']bipyrazinyl; 3-(3,4,5,6-tetrahydro-2H-[1,2']bipyrazinyl-6'-yloxymethyl)-benzonitrile; 6'-(2,5-difluoro-benzyloxy)-3,4,5,6-tetrahydro-,H-[1,2']bipyrazinyl; 5'-bromo-6'-(3-chloro-benzyloxy)-3,4,5,6-tetrahydro-2H-[1,2']bipyrazinyl; 6'-(3-bromo-benzyloxy)-3,4,5,6-tetrahydro-2H-[1,2']bipyrazinyl; 3-(3,4,5,6-tetrahydro-2H-[1,2']bipyrazinyl-6'-yloxymethyl)-phenylamine; 6'-(2-methyl-benzyloxy)-3,4,5,6-tetrahydro-2H-[1,2']bipyrazinyl; 6'-(3-chloro-benzyloxy)-5'-fluoro-3,4,5,6-tetrahydro-2H-[1,2']bipyrazinyl; 5'-chloro-6'-(3-chloro-benzyloxy)-3,4,5,6-tetrahydro-2H-[1,2']bipyrazinyl; 6'-(indan-(1S)-yloxy)-3,4,5,6-tetrahydro-2H-[1,2']bipyrazinyl; 6'-(3-methy-benzyloxy)-3,4,5,6-tetrahydro-2H-[1,2']bipyrazinyl; 6'-(3-chloro-benzyloxy)-3,4,5,6-tetrahydro-2H-[1,2']bipyrazinyl-3'-ylamine; 6'-(2-chloro-benzyloxy)-3,4,5,6-tetrahydro-2H-[1,2']bipyrazinyl; 6'-[2-(3-chloro-phenyl)-ethoxy]-3,4,5,6-tetrahydro-2H-[1,2']bipyrazinyl; 6'-(2-chloro-benzyloxy)-(2R)-methyl-3,4,5,6-tetrahydro-2H-[1,2']bipyrazinyl; 6'-(3,4-difluoro-benzyloxy)-3,4,5,6-tetrahydro-2H-[1,2']bipyrazinyl; 6'-(3,5-difluoro-benzyloxy)-3,4,5,6-tetrahydro-2H-[1,2']bipyrazinyl; (3-fluoro-benzyl)-(3,4,5,6-tetrahydro-2H-[1,2']bipyrazinyl-6'-yl)-amine; (3-chloro-benzyl)-(3,4,5,6-tetrahydro-2H-[1,2']bipyrazinyl-6'-yl)-amine; (3,5-difluoro-benzyl)-(3,4,5,6-tetrahydro-2H-[1,2']bipyrazinyl-6'-yl)-amine; 3-[(3,4,5,6-tetrahydro-2H-[1,2']bipyrazinyl-6'-ylamino)-methyl]-b enzonitrile; (2,5-difluoro-benzyl)-(3,4,5,6-tetrahydro-2H-[1,2']bipyrazinyl-6'-yl)-amine; (3,5-dichloro-benzyl)-(3,4,5,6-tetrahydro-2H-[1,2']bipyrazinyl-6'-yl)-amine; (3-chloro-benzyl)-(2-methyl-3,4,5,6-tetrahydro-2H-[1,2']bipyrazinyl-6'-yl)-amine; (3-fluoro-benzyl)-(2-methyl-3,4,5,6-tetrahydro-2H-[1,2']bipyrazinyl-6'-yl)-amine; (2-chloro-benzyl)-(3,4,5,6-tetrahydro-2H-[1,2']bipyrazinyl-6'-yl)-amine; (2-chloro-6-fluoro-benzyl)-(3,4,5,6-tetrahydro-2H-[1,2']bipyrazinyl-6'-yl)-amine; (2,3-difluoro-benzyl)-(3,4,5,6-tetrahydro-2H-[1,2']bipyrazinyl-6'-yl)-amine; N-(3-chloro-benzyl)-N-(3,4,5,6-tetrahydro-2H-[1,2']bipyrazinyl-6'-yl)-acetamide; 6'-(3-chloro-benzylsulfanyl)-3,4,5,6-tetrahydro-2H-[1,2']bipyrazinyl; or 6'-benzylsulfanyl-3,4,5,6-tetrahydro-2H-[1,2']bipyrazinyl.

Non-limiting examples of more preferred compounds include: 6'-benzyloxy-3,4,5,6-tetrahydro-2H-[1,2']bipyrazinyl; 6'-(3-chloro-benzyloxy)-3,4,5,6-tetrahydro-2H-[1,2']bipyrazinyl; 6'-(3-chloro-benzyloxy)-(2R)-methyl-3,4,5,6-tetrahydro-2H-[1,2']bipyrazinyl; 6'-(2-chloro-benzyloxy)-(2R)-methyl-3,4,5,6-tetrahydro-2H-[1,2']bipyrazinyl; 6'-(3-fluoro-benzyloxy)-(2R)-methyl-3,4,5,6-tetrahydro-2H-[1,2']bipyrazinyl; 6'-(2-fluoro-benzyloxy)-3,4,5,6-tetrahydro-2H-[1,2']bipyrazinyl; 6'-(3-fluoro-benzyloxy)-3,4,5,6-tetrahydro-2H-[1,2']bipyrazinyl; 6'-(3-chloro-benzyloxy)-2-ethyl-3,4,5,6-tetrahydro-2H-[1,2']bipyrazinyl; 6'-(2,5-difluoro-benzyloxy)-3,4,5,6-tetrahydro-2H-[1,2']bipyrazinyl; 6'-(2,5-dichloro-benzyloxy)-3,4,5,6-tetrahydro-2H-[1,2']bipyrazinyl; 6'-(3,5-difluoro-benzyloxy)-3,4,5,6-tetrahydro-2H-[1,2']bipyrazinyl; (3-fluoro-benzyl)-(3,4,5,6-tetrahydro-2H-[1,2']bipyrazinyl-6'-yl)-amine; (3-chloro-benzyl)-(3,4,5,6-tetrahydro-2H-[1,2']bipyrazinyl-6'-yl)-amine; (3,5-difluoro-benzyl)-(3,4,5,6-tetrahydro-2H-[1,2']bipyrazinyl-6'-yl)-amine; (3,5-dichloro-benzyl)-(3,4,5,6-tetrahydro-2H-[1,2']bipyrazinyl-6'-yl)-amine; (2-chloro-6-fluoro-benzyl)-(3,4,5,6-tetrahydro-2H-[1,2']bipyrazinyl-6'-yl)-amine; 6'-(3-chloro-benzyloxy)-3,4,5,6-tetrahydro-2H-[1,2']bipyrazinyl 4'-oxide; N-(3-chloro-benzyl)-N-(3,4,5,6-tetrahydro-2H-[1,2']bipyrazinyl-6'-yl)-acetamide; 6'-(3-chloro-benzylsulfanyl)-3,4,5,6-tetrahydro-2H-[1,2']bipyrazinyl; or 6'-(indan-(1S)-yloxy)-3,4,5,6-tetrahydro-2H-[1,2']bipyrazinyl.

Even more preferred compounds of Formula (IIB) include: 6'-benzyloxy-3,4,5,6-tetrahydro-2H-[1,2']bipyrazinyl; 6'-(3-chloro-benzyloxy)-3,4,5,6-tetrahydro-2H-[1,2']bipyrazinyl; 6'-(3-fluoro-benzyloxy)-3,4,5,6-tetrahydro-2H-[1,2']bipyrazinyl; 6'-(3-chloro-benzyloxy)-3,4,5,6-tetrahydro-2H-[1,2']bipyrazinyl 4'-oxide; (3-fluoro-benzyl)-(3,4,5,6-tetrahydro-2H-[1,2']bipyrazinyl-6'-yl)-amine; 6'-(3-chloro-benzyloxy)-(2R)-methyl-3,4,5,6-tetrahydro-2H-[1,2']bipyrazinyl; 6'-(3,5-difluoro-benzyloxy)-3,4,5,6-tetrahydro-2H-[1,2']bipyrazinyl; 6'-(2,5-difluoro-benzyloxy)-3,4,5,6-tetrahydro-2H-[1,2']bipyrazinyl; or 6'-(indan-(1S)-yloxy)-3,4,5,6-tetrahydro-2H-[1,2']bipyrazinyl.

Other suitable 5-HT2C receptor agonists and antagonists can also be found, for example, in patent applications EP 863136, EP 657426, EP655440, EP 572863, WO 98/30548, WO 98/56768, WO 99/43647, WO 99/43647, WO 99/58490, WO 00/12475, WO 00/12481, WO 00/12482, WO 00/12502, WO 00/12510, WO 00/17170, WO 00/28993, WO 00/35922, WO 00/44737, WO 00/76984, WO 00/77001, WO 00/77002, WO 0077010 (preferably Examples 128, 149), WO 01/12602, WO 01/12603, WO 01/66548, WO 01/72752 WO 01/83487, WO 02/48124, WO 02/051844, WO 02/059124, WO 02/059129, WO 02/072584, WO 02/074746, WO 03/004501, WO 03/014118, WO 03/014125, WO 03/024976, WO 03/033497, WO 03/057161, WO 03/057213, WO 03/057673, WO 03/057674, US 6,593,330, US 2004/014767, WO 04/000830. Another suitable 5-HT2C receptor agonist may be Ro-600175 (Jenk, F. et al (1998) Eur. J. Neuropharmacol. 8, 161-168; Dekeyne, A. et al (1999) Neuropharmacology 38, 415-423). Other suitable 5HT2C agonists for use in the invention are disclosed in Isaac, M. et al (2000) Bioorg. Med. Chem Lett. 10, 919-921.

The compounds exemplified in a recent review of 5HT2C agonists are also included for use in the invention (Bishop,M.J & Nilsson,B.M. (2003) Expert Opin.Ther. Patents 13, 1691-1705).

A method of enhancing urethral tone and reducing undesirable urine leakage in a healthy person, e.g. alleviating urine leakage often occurring in women during the first year after child-birth, comprising administering a 5-HT2C receptor agonist is a further aspect of the invention.

Yet a further aspect of the invention is a method of screening for compounds useful for treating incontinence, preferably mixed incontinence and stress urinary incontinence, comprising screening compounds for agonist activity against 5-HT2C receptors, and selecting compounds with an EC₅₀ of less than 1µM, preferably with an EC₅₀ of less than 100nM, more preferably with an EC₅₀ of less than 10nM, even more preferably with an EC₅₀ of less than 1nM.

Another aspect of the invention is a process for providing a medicament for the treatment of incontinence, preferably for the treatment of mixed incontinence and stress urinary incontinence, comprising the following steps:
(a) testing compounds in an assay suitable for detecting activation of 5-HT2C receptors;
(b) selecting a compound with an EC₅₀ of less than 1µM;
(c) formulating a compound with the same structure as that selected in step (b), or a pharmaceutically acceptable salt thereof, with a pharmaceutically acceptable carrier or excipient; the process may also comprise the additional steps of:
(d) packaging the formulation of step (c); and
(e) making the package of step (d) available to a patient suffering from incontinence.

Preferably, the compound selected in step (b) will have an EC₅₀ of less than 100nM, more preferably it will have an EC₅₀ of less than 10nM, even more preferably it will have an EC₅₀ of less than 1 nM.

Yet another aspect of the invention is a process for providing a medicament for the treatment of incontinence, preferably for the treatment of mixed incontinence and stress urinary incontinence, comprising the following steps:
(a) testing compounds in an assay, measuring the agonist-stimulated second messenger response of 5-HT2C receptors;
(b) selecting a compound with an EC₅₀ of less than 1µM;
(c) formulating a compound with the same structure as that selected in step (b), or a pharmaceutically acceptable carrier or excipient; the process may also comprise the additional steps of:
(d) packaging the formulation of step (c); and
(e) making the package of step (d) available to a patient suffering from incontinence.

Preferably, the assay in step (a) measures a transient rise in intracellular calcium in 5-HT2C receptor-expressing cells in response to a 5-HT2C receptor agonist; even more preferably, the transient rise in intracellular calcium is measured by fluorescence techniques, e.g. using calcium-sensitive fluorescent dyes such as Fluo-3 or Fluo-4. Preferably, the compound selected in step (b) will have an EC₅₀ of less than 100 nM, more preferably, it will have an EC₅₀ of less than 10nM, even more preferably it will have an EC₅₀ of less than 1 nM.

Another aspect of the invention is a process for preparing a medicament for the treatment of incontinence, preferably for the treatment of stress urinary incontinence, comprising the steps of (a) testing compounds in an assay suitable for detecting stimulation of 5-HT2C receptor, or testing compounds in an assay, measuring the agonist stimulated second messenger response of 5-HT2C receptor; (b) identifying one or more compounds capable of agonising 5-HT2C receptor with an EC₅₀ of less than 1µM; and (c) preparing a quantity of those one or more identified compounds. Preferably, the compound(s) selected in step (b) will have an EC₅₀ of less than 100 nM, more preferably it/they will have an EC₅₀ of less than 10nM, even more preferably it/they will have an EC₅₀ of less than 1 nM.

Another aspect of the invention is a method of preparing a composition for treating incontinence, preferably for treating stress urinary incontinence, which comprises:
(a) identifying a compound which specifically binds to 5-HT2C receptor by a method which comprises contacting cells expressing 5-HT2C receptor or membranes prepared from such cells with a radiolabelled 5-HT2C receptor ligand in the presence or absence of a test compound, measuring the radioactivity bound to the cells or membranes, comparing the radioactivity bound to the cells or membranes in the presence and absence of test compound, whereby a compound which causes a reduction in the radioactivity bound is a compound specifically binding to 5-HT2C receptor; and
(b) admixing said compound with a carrier.

Yet another aspect of the invention is a method of preparing a composition for treating incontinence, preferably for treating stress urinary incontinence, which comprises:
(a) identifying a compound which specifically activates 5-HT2C receptor by a method which comprises separately contacting cells expressing 5-HT2C receptor on their surface and producing a second messenger response in response to a 5-HT2C receptor agonist, or a membrane preparation of such cells, with the compound, under conditions suitable for activation of 5-HT2C receptor, and measuring the second messenger response, with an increase of the second messenger response after administration of the compound indicating that the compound is an agonist of the 5-HT2C receptor; and
(b) admixing said compound with a carrier.

The invention relates to the use of a 5-HT2C receptor agonist for the treatment of incontinence alone, or in combination with one or more other agents such as alpha-adrenergic receptor agonists or other sympathomimetic drugs.

A 5-HT2C receptor agonist is a compound which binds to the 5-HT2C receptor and activates it, producing a pharmacological response. The term is meant to include a partial or a full agonist. A partial agonist is a compound which is unable to produce maximal activation of the receptor and so has an intrinsic efficacy of <1. In a functional sense such a compound would be unable to evoke a response of equal or greater magnitude than a full agonist (for example, but not limited to, 5-HT) in a functional assay system under investigation no matter how high a concentration is applied to that assay system. An antagonist of the 5-HT2C receptor is a compound which attenuates or blocks the effect of an agonist of the receptor.

Reference to an antagonist, an agonist or an inhibitor shall at all times be understood to include all active forms of such agents, including the free form thereof (e.g. the free and/or base form) and also all pharmaceutically acceptable salts, polymorphs, hydrates, silicates, stereo-isomers (e.g. diastereoisomers and enantiomers) and so forth. Active metabolites of any of the compounds, in any form, are also included.

For the avoidance of doubt, the term "compound" may refer to a chemical or biological agent, and includes, for example, antibodies, antibody fragments, other proteins, peptides, sugars, any organic or inorganic molecules. Compounds that may be used for screening include, but are not limited to, peptides such as, for example, soluble peptides, including but not limited to members of random peptide libraries; (see, e.g., Lam et al. (1991) Nature 354, 82-84; Houghten et al. (1991) Nature 354, 84-86), and combinatorial chemistry-derived molecular library made of D- and/or L-configuration amino acids, phosphopeptides (including, but not limited to, members of random or partially degenerate, directed phosphopeptide libraries; see, e.g., Songyang et al. (1993) Cell 72, 767-778), antibodies (including, but not limited to, polyclonal, monoclonal, humanized, anti-idiotypic, chimeric or single chain antibodies, and Fab, F(ab')₂ and Fab expression library fragments, and epitope-binding fragments thereof), and small organic or inorganic molecules.

"Potency" as used herein is a measure of how effective a compound is at producing the desired response and can be expressed in terms of the concentration which produces a particular level of the response attainable. Affinity as used herein is a measure of how well a compound binds to or becomes associated with a receptor. The affinity of a compound can be determined in a binding assay as described in Example 2 herein, and affinity in this context will refer to the IC₅₀ of the compound, i.e. to the concentration inhibiting 50% of the labelled compound from binding to the receptors, or to the Kᵢ, which is the dissociation constant of the compound. The potency or efficacy of a compound can be determined in a functional assay such as an assay measuring a rise in intracellular calcium upon stimulation of the receptor, e.g. using calcium-sensitive fluorescent dyes such as Fluo-3, Fluo-4 or Indo-1 (Example 3 herein) or an anaesthetised animal model to test the effect of compounds on micturition or urine leakage as described in Example 1 herein. The potency/efficacy in this case could refer to the EC₅₀ of the compound, i.e. the concentration which shows 50% of the maximal response to serotonin (or any other known full agonist of the 5-HT2C receptor).

"Selectivity" as used herein is a measure of the relative potency of a drug between two receptor subtypes for the same endogenous ligand. This can be determined in binding assays, e.g. as described in Example 2 herein, or in functional assays, e.g. as described in Example 3 herein.

Particular formulations of the compounds for either oral delivery or for topical application (creams, gels) are included in the invention. Also included in the invention are for example, intravesicle, anal or vaginal formulations.

The suitability of the 5-HT2C receptor agonists can be readily determined by evaluation of their potency/efficacy and selectivity using methods such as those disclosed herein, followed by evaluation of their toxicity, pharmacokinetics (absorption, metabolism, distribution and elimination), etc in accordance with standard pharmaceutical practice. Suitable compounds are those that are potent and selective, have no significant toxic effect at the therapeutic dose, and preferably are bioavailable following oral administration.

Oral bioavailablity refers to the proportion of an orally administered drug that reaches the systemic circulation. The factors that determine oral bioavailability of a drug are dissolution, membrane permeability and hepatic clearance. Typically, a screening cascade of firstly *in vitro* and then *in vivo* techniques is used to determine oral bioavailablity.

Dissolution, the solubilisation of the drug by the aqueous contents of the gastrointestinal tract (GIT), can be predicted from in vitro solubility experiments conducted at appropriate pH to mimic the GIT. Preferably the 5-HT2C receptor agonists have a minimum solubility of 50µg/ml. Solubility can be determined by standard procedures known in the art such as described in Lipinski CA et al.; Adv. Drug Deliv. Rev. 23(1-3), 3-25, 1997.

Membrane permeability refers to the passage of a compound through the cells of the GIT. Lipophilicity is a key property in predicting this and is determined by *in vitro* Log D_{7.4} measurements using organic solvents and buffer. Preferably the 5-HT2C receptor agonists have a Log D_{7.4} of -2 to +4, more preferably -1 to +3. The Log D can be determined by standard procedures known in the art such as described in Stopher, D and McClean, S; J. Pharm. Pharmacol. 42(2), 144, 1990.

Cell monolayer assays such as Caco2 add substantially to prediction of favourable membrane permeability in the presence of efflux transporters such as P-glycoprotein, so-called Caco2 flux. Preferably, the 5-HT2C receptor agonists have a Caco2 flux of greater than 2x10⁻⁶cms⁻¹, more preferably greater than 5x10⁻⁶cms⁻¹. The Caco2 flux value can be determined by standard procedures known in the art such as described in Artursson, P and Magnusson, C; J. Pharm. Sci, 79(7), 595-600, 1990.

Metabolic stability addresses the ability of the GIT to metabolise compounds during the absorption process or the liver to do so immediately post-absorption: the first pass effect. Assay systems such as microsomes, hepatocytes etc are predictive of metabolic lability. Preferably 5-HT2C receptor agonists show metabolic stability in the assay system that is commensurate with an hepatic extraction of less then 0.5. Examples of assay systems and data manipulation are described in Obach, RS; Curr. Opin. Drug Disc. Devel. 4(1), 36-44, 2001 and Shibata, Y et al.; Drug Met. Disp. 28(12), 1518-1523, 2000.

Because of the interplay of the above processes, further support that a drug will be orally bioavailable in humans can be gained by *in vivo* experiments in animals.

Absolute bioavailability is determined in these studies by administering the compound separately or in mixtures by the oral route. For absolute determinations (% orally bioavailable) the intravenous route is also employed. Examples of the assessment of oral bioavailability in animals can be found in Ward, KW et al.; Drug Met. Disp. 29(1), 82-87, 2001; Berman, J et al.; J. Med. Chem. 40(6), 827-829, 1997 and Han KS and Lee, MG; Drug Met. Disp. 27(2), 221-226, 1999.

The compounds of the invention can be administered alone but will generally be administered in admixture with a suitable pharmaceutical excipient, diluent or carrier selected with regard to the intended route of administration and standard pharmaceutical practice.

For example, the compounds of the invention can be administered orally, buccally or sublingually in the form of tablets, capsules, multi-particulates, gels, films, ovules, elixirs, solutions or suspensions, which may contain flavouring or colouring agents, for immediate-, delayed-, modified-, sustained-, pulsed- or controlled-release applications. The compounds of the invention may also be administered as fast-dispersing or fast-dissolving dosage forms or in the form of a high energy dispersion or as coated particles. Suitable formulations may be in coated or uncoated form, as desired.

Such solid pharmaceutical compositions, for example, tablets, may contain excipients such as microcrystalline cellulose, lactose, sodium citrate, calcium carbonate, dibasic calcium phosphate, glycine and starch (preferably corn, potato or tapioca starch), disintegrants such as sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

The following formulation examples are illustrative only and are not intended to limit the scope of the invention. Active ingredient means a compound of the invention.

### Formulation 1:

A tablet is prepared using the following ingredients :
Active ingredient (50mg) is blended with cellulose (microcrystalline), silicon dioxide, stearic acid (fumed) and the mixture is compressed to form tablets.

### Formulation 2:

An intravenous formulation may be prepared by combining active ingredient (100mg) with isotonic saline (1000ml)

The tablets are manufactured by a standard process, for example, direct compression or a wet or dry granulation process. The tablet cores may be coated with appropriate overcoats.

Solid compositions of a similar type may also be employed as fillers in gelatin or HPMC capsules. Preferred excipients in this regard include lactose, starch, a cellulose, milk sugar or high molecular weight polyethylene glycols. For aqueous suspensions and/or elixirs, the 5-HT2C receptor agonist may be combined with various sweetening or flavouring agents, colouring matter or dyes, with emulsifying and/or suspending agents and with diluents such as water, ethanol, propylene glycol and glycerin, and combinations thereof.

Modified release and pulsatile release dosage forms may contain excipients such as those detailed for immediate release dosage forms together with additional excipients that act as release rate modifiers, these being coated on and/or included in the body of the device. Release rate modifiers include, but are not exclusively limited to, hydroxypropylmethyl cellulose, methyl cellulose, sodium carboxymethylcellulose, ethyl cellulose, cellulose acetate, polyethylene oxide, Xanthan gum, Carbomer, ammonio methacrylate copolymer, hydrogenated castor oil, carnauba wax, paraffin wax, cellulose acetate phthalate, hydroxypropylmethyl cellulose phthalate, methacrylic acid copolymer and mixtures thereof. Modified release and pulsatile release dosage forms may contain one or a combination of release rate modifying excipients. Release rate modifying excipients may be present both within the dosage form i.e. within the matrix, and/or on the dosage form, i.e. upon the surface or coating.

Fast dispersing or dissolving dosage formulations (FDDFs) may contain the following ingredients: aspartame, acesulfame potassium, citric acid, croscarmellose sodium, crospovidone, diascorbic acid, ethyl acrylate, ethyl cellulose, gelatin, hydroxypropylmethyl cellulose, magnesium stearate, mannitol, methyl methacrylate, mint flavouring, polyethylene glycol, fumed silica, silicon dioxide, sodium starch glycolate, sodium stearyl fumarate, sorbitol, xylitol. The terms dispersing or dissolving as used herein to describe FDDFs are dependent upon the solubility of the drug substance used i.e. where the drug substance is insoluble a fast dispersing dosage form can be prepared and where the drug substance is soluble a fast dissolving dosage form can be prepared.

The compounds of the invention can also be administered parenterally, for example, intracavernouslly, intravenously, intra-arterially, intraperitoneally, intrathecally, intraventricularly, intraurethrally, intrasternally, intracranially, intramuscularly or subcutaneously, or they may be administered by infusion or needleless injection techniques. For such parenteral administration they are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

The following dosage levels and other dosage levels herein are for the average human subject having a weight range of about 65 to 70kg. The skilled person will readily be able to determine the dosage levels required for a subject whose weight falls outside this range, such as children and the elderly.

The dosage of the combination of the invention in such formulations will depend on its potency, but can be expected to be in the range of from 1 to 500mg of 5-HT2C receptor agonist for administration up to three times a day. A preferred dose is in the range 10 to 100mg (e.g. 10, 25, 50 and 100mg) of 5-HT2C receptor agonist which can be administered once, twice or three times a day (preferably once). However the precise dose will be as determined by the prescribing physician and will depend on the age and weight of the subject and severity of the symptoms.

For oral and parenteral administration to human patients, the daily dosage level of a compound of the invention will usually be from to 5 to 500mg/kg (in single or divided doses).

Thus tablets or capsules may contain from 5mg to 250mg (for example 10 to 100mg) of the compound of the invention for administration singly or two or more at a time, as appropriate. The physician in any event will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention. The skilled person will appreciate that the compounds of the invention may be taken as a single dose as needed or desired (i.e. prn). It is to be appreciated that all references herein to treatment include acute treatment (taken as required) and chronic treatment (longer term continuous treatment).

The compounds of the invention can also be administered intranasally or by inhalation and are conveniently delivered in the form of a dry powder inhaler or an aerosol spray presentation from a pressurised container, pump, spray, atomiser or nebuliser, with or without the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A [trade mark]) or 1,1,1,2,3,3,3-heptafluoropropane (HFA 227EA [trade mark]), carbon dioxide or other suitable gas. In the case of a pressurised aerosol, the dosage unit may be determined by providing a valve to deliver a metered amount. The pressurised container, pump, spray, atomiser or nebuliser may contain a solution or suspension of the active compound, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of the compounds of the invention and a suitable powder base such as lactose or starch.

Aerosol or dry powder formulations are preferably arranged so that each metered dose or "puff" contains from 1µg to 50mg of a compound of the invention for delivery to the patient. The overall daily dose with an aerosol will be in the range of from 1µg to 50mg which may be administered in a single dose or, more usually, in divided doses throughout the day.

Alternatively, the compounds of the invention can be administered in the form of a suppository or pessary, or they may be applied topically in the form of a gel, hydrogel, lotion, solution, cream, ointment or dusting powder. The compounds of the invention may also be dermally or transdermally administered, for example, by the use of a skin patch, depot or subcutaneous injection. They may also be administered by the pulmonary or rectal routes.

For application topically to the skin, the compounds of the invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The compounds of the invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in published international patent applications WO91/11172, WO94/02518 and WO98/55148.

Oral administration of the compounds of the invention is a preferred route, being the most convenient. In circumstances where the recipient suffers from a swallowing disorder or from impairment of drug absorption after oral administration, the drug may be administered parenterally, sublingually or buccally.

### Examples

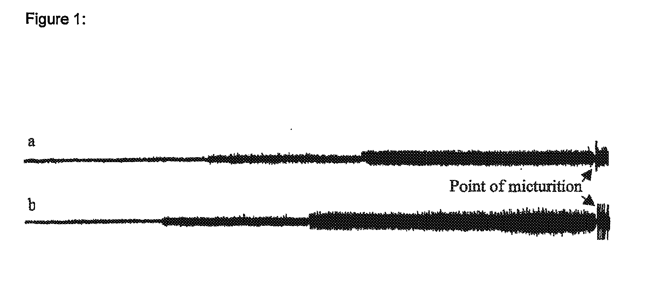

The examples below are carried out using standard techniques, which are well-known and routinely used by those skilled in the art; the examples illustrate but do not limit the invention. Reference is made to Figure 1.

Figure 1 is a trace showing the effects of Way 161503 on EUS EMG activity during normal bladder filling

### Example 1a: Effects of 5-HT2C receptor agonists on urethral function in the guinea-pig.

In order to explore what subtypes of 5-HT receptors may be involved in enhancing the sacral spinal drive to the EUS, the effects of m-CPP (Sigma Aldrich Product number C-5554), MK-212 (Tocris Cat No 0941), YM-348 (WO 01/83487), Ro 60-0175 (Tocris Cat No 1854), WAY-161503 (Tocris Cat No 1801), and Example 1-N in WO 03/000663, which have been reported to be 5-HT2C receptor agonists, were investigated in an anaesthetised guinea pig model specifically designed to measure urethral continence mechanisms and leak point pressure using two paradigms. Firstly the activity of urethral striated musculature in response to increased abdominal pressure and so the pressure required to induce leak and secondly, the response of urethral striated musculature during normal bladder filling so as to maintain continence, as measured by changes in electromyographic activity of this continence maintaining sphincter.

### Methods:

Experiments were performed in adult female guinea pigs, weighing 620-707 g. All animals were initially anaesthetised with halothane (4%), carried in oxygen (3-4L min⁻¹) and were maintained at an appropriate surgical plane with urethane (25% w/v; 0.5ml 100g⁻¹ body weight). The trachea, a jugular vein and a carotid artery were cannulated for respiratory ventilation, injection of test compound and monitoring of blood pressure, respectively. A midline laporatomy was performed to expose the urinary bladder and a cystometry tube was inserted through a small incision in the dome of the bladder and secured in place. The abdominal wound was closed tightly around the externalised cystometry tube, which, in turn, was connected to an infusion pump and pressure transducer, for filling the bladder and for recording intravesical pressure, respectively. Electromyographic (EMG) wire leads were inserted into the EUS striated muscle layer opposed to the dorsal surface of the symphysis pubis. The EMG leads were connected to an appropriate amplification and electrical filter system and changes in EUS electrical activity were displayed on an oscilloscope and recorded through a spike processor system.

Following a 30 min post surgery stabilisation period, the bladder was filled at a rate of 150 µl min⁻¹ with physiological saline (room temperature), until initiation of a micturition reflex or leak was observed. Following micturition or leak, the bladder was drained via the externalised cystometry tube. Bladder filling was repeated at least 3 times in order to establish a mean bladder threshold capacity for initiation of micturition or leak. Additionally, EUS EMG activity and intravesical pressures were recorded throughout bladder filling. Subsequently 1 of 2 protocols was carried out.
1. Once a threshold bladder volume, which induced micturition or leak, was established, 75% of this volume was used for the next stage of the experiment. The bladder was filled (150 µl min⁻¹) to 75% of threshold volume with physiological saline and, through the use of a specially constructed frame, weights were positioned on the ventral surface of the abdomen of the animal just rostral to the position of the bladder. Starting at 50g, then 60g and then at increasing increments of 20g, weights were placed on the animal's abdomen until micturition/leakage of fluid was observed. EUS EMG activity and intravesical pressure were recorded while weights were applied to the abdomen. The main parameters investigated were minimum abdominal weight required to induce micturition or leak at 75% of bladder threshold volume, and maximum EUS EMG activity at micturition or leak. Test drug or vehicle was injected intravenously immediately after the bladder was filled to 75% of threshold volume, and 60-120 sec before applying the first abdominal weight (50g).
2. Test drug or vehicle was injected intravenously and bladder filling was reinitiated (150 µl min⁻¹) until micturition occurred, the bladder was then drained via the externalised cystometry tube before addition of an increased dose of test compound and further bladder filling were attempted. Bladder pressure and maximum EUS EMG activity were recorded throughout the filling phase such that compound induced increases in EUS EMG activity during normal filling could be recorded

Agonists were tested at doses between 0.1 and 3.0 mg kg⁻¹. In addition the effect of the reported 5-HT2C selective antagonist SB 242084 (Sigma, Cat-No: S-8061) (1 mg kg⁻¹) on agonist-induced effects was investigated in some animals.

### Results

Administration of 5-HT2C agonists increased the abdominal weight required to induce micturition or leak in anaesthetised guinea pigs (Table 1, for purposes of illustration only results obtained with m-CPP are presented). In conjunction with this rise in abdominal weight, EUS EMG activity also increased in a dose dependent manner (Table 1, for purposes of illustration only results obtained with m-CPP are presented).

**Table 1: The effect of m-CPP on abdominal weight inducing micturition or leak**

| The 5-HT2C agonist, m-CPP, was tested at 0.3, 1.0 and 3.0 mg kg⁻¹. Results are given as % of control response. Thus at the 3.0 mg kg⁻¹ level, m-CPP increased the weight required to induce micturition or leak by 66% compared with controls, with a corresponding increase in EUS EMG activity of 56%. Vehicle alone had no effect. | | | |
|---|---|---|---|
| **m-CPP Dose (mg kg⁻¹ iv.)** | **n** | **% of Control Abdominal Weight Inducing Micturition or Leak** | **% of Control EUS EMG Activity** |
| **0.3** | 2 | 128 ± 6 | 110 ± 43 |
| **1.0** | 3 | 139 ± 12 | 135 ± 10 |
| **3.0** | 2 | 166 ± 9 | 156 ± 18 |

During normal bladder filling (150 µl min⁻¹) the EUS EMG activity (a measure of the contractile activity of the urethral sphincter) increased gradually until micturition (voiding) occurred (Fig 1 a), after which time activity returned to baseline level. Repeated filling and micturition cycles produced similar increases in EUS EMG activity. Subsequent administration of 5-HT2C agonists and repeated normal bladder filling resulted in an increase in EUS EMG activity above that recorded in the absence of drug or on administration of vehicle alone (Fig 1b, for purposes of illustration only results obtained with Way 161503 are presented). In drug free conditions (a) as the bladder is filled (150 µl min⁻¹) activity of the urethral striated muscle (EUS EMG) increases in order to maintain continence. In the presence of Way 161503 (0.3 mg kg⁻¹) EUS EMG activity was potentiated under identical bladder filling conditions, additionally increases in activity occurred at lower bladder volumes than in drug free conditions (b). In those animals tested, the selective 5-HT2C receptor antagonist SB 242084 abolished the effects of applied 5HT2c agonists.

### Example 1b: Effect of MK-212 on leak point pressure in ovariectomised, birth traumatised female rats.

In order to evaluate whether the effects of 5-HT2C agonists on urethral function was present in species other than the guinea pig, leak point pressure was measured in the absence and presence of MK-212 in a known model of urethral function in the rat (Lin A.S. et al. (1998) Urology 52:143-51; Sievert K.D.et al. (2001) J Urol. 166, 311-317; Resplande J et al (2002) J Urol. 168, 323-30), namely ovariectomised, birth traumatised rats.

### Methods

Ovariectomy and simulated birth trauma were carried out on 8 female Sprague Dawley rats as described previously (Lin A.S. et al. (1998) Urology 52:143-51; Sievert K.D.et al. (2001) J Urol. 166, 311-317; Resplande J et al (2002) J Urol. 168, 323-30). Six weeks subsequent to recovery all animals underwent investigation of leak point pressure, initially rats were anaesthetised with halothane (4%), carried in oxygen (3-4L min⁻¹) and were maintained at an appropriate surgical plane with urethane (25% w/v; 0.5ml 100g⁻¹ body weight). The trachea, a jugular vein and a carotid artery were cannulated for respiratory ventilation, injection of test compound and monitoring of blood pressure, respectively. A midline laporatomy was performed to expose the urinary bladder and a cystometry tube was inserted through a small incision in the dome of the bladder and secured in place. The abdominal wound was closed tightly around the externalised cystometry tube, which, in turn, was connected to an infusion pump and pressure transducer, for filling the bladder and for recording intravesical pressure, respectively. Baseline leak point pressures (LPP) were established by slow elevation of a saline containing bag connected to the bladder and pressure transducer via the cystometry tube. LPP was defined as the bladder pressure at the point where fluid was first noted at the urethral meatus. Between 4 and 7 baseline readings were made before iv bolus administration of MK-212 (0.1 mg kg⁻¹). Following a 2 min delay to allow for distribution LPP measurements were repeated in an identical manner to that described above, between 3 and 8 measures were taken.

### Results

Application of MK-212 (0.1 mg kg⁻¹) increased LPP pressure in 7 of 8 animals (Table 2). The calculated mean increases in LPP per animal ranged from 3.6 to 10.9 mmHg with a mean increase of 7.1 ± 2.6 (mean ± standard deviation, N=7).

**Table 2: The effect of MK-212 on leak point pressure in ovariectomised, birth traumatised rats**

| **Animal No** | **LPP (mean ± SD, mmHg)** | | **Increase (mmHg)** |
|---|---|---|---|
| | **Baseline** | **MK-212 (0.1 mg/kg)** | |
| 1 | 22.6 (2.3) | 33.5 (1.6) | 10.9 |
| 2 | 16.3 (1.3) | 23.0 (2.6) | 6.7 |
| 3 | 20.4 (2.8) | 30.6 (3.8) | 10.2 |
| 4 | 17.9 (5.2) | 23.4 (3.4) | 5.5 |
| 5 | 14.8 (0.0) | 21.8 (1.7) | 7.0 |
| 6 | 17.8 (1.0) | 18.2 (1.5) | 0.4 |
| 7 | 24.0 (1.2) | 27.6 (1.5) | 3.6 |
| 8 | 24.7 (0.6) | 30.7 (1.2) | 6.0 |

The effect elicited in these animal models, i.e. the increase in urethral striated muscle activity and leak point pressure, would be beneficial in stress urinary incontinence and the stress component of mixed urinary incontinence.

### Example 2: Ligand binding assay for 5-HT2C receptor

Affinity of compounds at the serotonin 5-HT2C receptor is determined by competition binding in Swiss 3T3 mouse fibroblasts (available from the American Type Culture Collection (ATCC), Manassas, VA), transfected with a plasmid driving the expression of the human 5-HT2C receptor in mammalian cells, against ³H-5-HT. The method is adapted from Roth et al, (1992), J. of Pharm and Exp. Therap. 260(3), 1362-1365. Cells are grown in DMEM high glucose medium, harvested, homogenised, centrifuged, and resuspended in 50mM Tris-HCl. They are incubated at 37°C for 15 minutes, centrifuged, and then resuspended into assay buffer (50mM Tris-HCl, 4mM CaCl₂, 0.1% ascorbic acid, and 100µM pargyline at pH 7.7) at 100 volumes per gram. Assay tubes contained 25µl of 10nM ³H-5-HT (1nM final concentration), and 25µl vehicle (assay buffer), blank (10mM mianserin) or test compound (10x final concentration). 200µl of cell homogenate was added to each tube, vortexed, and incubated for 30 minutes at 37°C. Samples are then rapidly filtered under vacuum with a Skatron cell harvester (available from Molecular Devices Corporation, Sunnyvale, CA) using GF/B filters presoaked in 0.5% polyethyleneimine (PEI), and washed with 2x 5ml cold Tris-HCl. Filtermats are removed and counted in a Wallac Betaplate Counter (available from Perkin Elmer Life Sciences, Gaithersburg, MD). Percent inhibition of specific binding by test compounds is used to calculate the Kᵢ, or extrapolate concentration of test compound necessary to inhibit 50% of the total specific binding for each compound (IC₅₀).

### Example 3: Functional assay

Swiss 3T3 cells expressing human 5-HT2C receptors are seeded at a density of 12,500 cells/well in 384 well black/clear collagen-coated plates. 48 hours later the cells are loaded with the calcium sensitive dye, Fluo-4-AM (available from Molecular Probes; 4µM dissolved in DMSO containing pluronic acid) in serum-free DMEM in the presence of probenicid (2.5mM) for 75 minutes at 37°C in a CO₂ incubator. Unincorporated dye is removed by washing 3 times with HEPES-buffered saline containing probenicid (2.5mM) using a Skatron^{™} cell washer (final volume 30µl).

Plates are then placed in a fluorometric imaging plate reader (FLIPR 384, available from Molecular Devices Corporation) individually, and fluorescence measurements are taken every 2 seconds over an 85 seconds period. Test compound additions are made simultaneously to all 384 wells after 20 seconds of baseline recording. Concentration response curves are generated using Graphpad PrismTM (available from GraphPad Software Inc., San Diego, CA) and agonist efficacies are generated as % of the response to 10mM 5-HT (considered as 100%). Estimation of antagonist potencies (functional Kis) are generated by measuring inhibition of the test compound response to 5-HT (10nM) and applying the Cheng Prusoff equation.

The skilled person will be able to adapt the above ligand binding and functional assays for other 5-HT receptor subtypes; similar assays for alpha-adrenergic receptors can be found in the literature. For example, a functional assay for 5HT2A, 5HT2B and 5HT2C receptors can be found in Porter,R.H.P. et al (1999) Brit. J. Pharmacol. 128, 13-20; assays for 5HT2C and a multitude of other receptors can also be found in Martin, J.R. (1998) J. Pharmacol. Exp. Ther. 286, 913-924 or in Kimura, Y et al (2004) Eur. J. Pharmacol 483, 37-43).

## Claims

1. Use of an agonist for 5-HT2C receptor in the manufacture of a medicament for the treatment of stress urinary incontinence, provided the agonist is not 1-[6-chloro-5-(trifluoromethyl)-2-pyridinyl]piperazine (Org-12962).

2. The use of claim 1, wherein the agonist for 5-HT2C receptor is MK212, Ro-60-0175, WAY-161503, or YM-348, or a pharmaceutical acceptable salt thereof.

3. The use of claim 1, wherein the 5-HT2C receptor agonist is a compound of formula (IB): 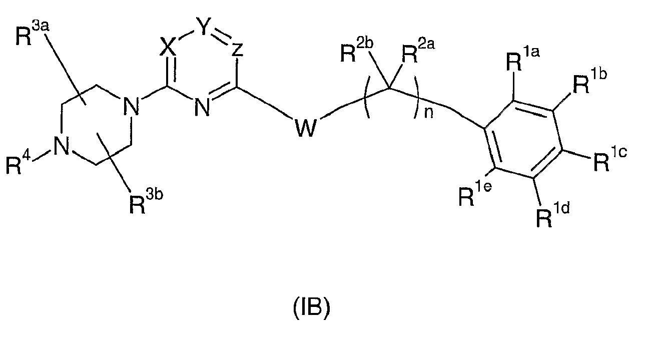 wherein:
X and Y are CR and Z is N, or X is N and Y and Z are CR, where R for each occurrence is hydrogen, halogen, (C1-C4)alkyl, amino, or (C1-C4)alkylamino;
W is oxy, thio, amino, (C1-C4)alkylamino, or acetylamino;
R^{1a}, R^{1b}, R^{1c}, R^{1d} and R^{1e} are each independently hydrogen, halogen, nitro, cyano, amino, (C1-C4)alkyl, halo-substituted (C1-C4)alkyl, (C1-C4)alkoxy, halo-substituted (C1-C4) alkoxy, -C(O) NH₂, R^{1a} and R^{1b} taken together form a five-or six-membered, aromatic or partially or fully saturated fused ring, or R^{1a} taken together with R^{2a} or R^{2b} forms a five-or six-membered, fully saturated, fused ring;
R^{2a} and R^{2b} are each independently hydrogen, (C1-C4)alkyl, partially or fully saturated (C3-C6) cycloalkyl, or one of which taken together with Reforms a five-or six-membered, fully saturated fused ring;
n is 0, 1, or 2;
R^{3a} and R^{3b} are each independently hydrogen, (C1-C4)alkyl, (C1-C4)alkyl substituted with hydroxy, fluoro, or (C1-C4) alkoxy;
R⁴ is hydrogen, hydroxy, (C1-C4)alkyl, (C1-C4)alkyl substituted with hydroxy or cyano, (C1-C4)alkylcarbonyl, (C1-C4) alkoxy, (C1-C4) alkoxy-carbonyl, or (C3-C4) alkenyl;
a nitrogen oxide thereof, a prodrug of the compound or the nitrogen oxide; a pharmaceutical acceptable salt of the compound, the nitrogen oxide, or the prodrug, or a solvate or hydrate of the compound, the nitrogen oxide, the prodrug, or the salt.

4. The use of claim 1, wherein the 5HT2C receptor agonist is a compound of formula (IC): 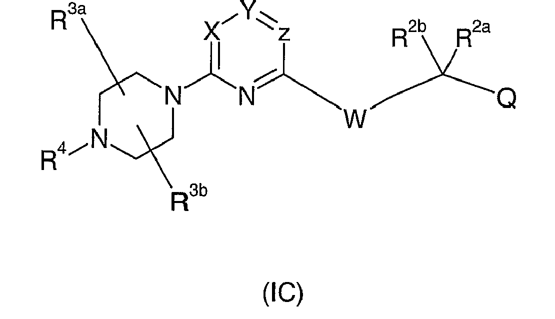 wherein:
X and Y are CR and Z is N, or X is N and Y and Z are CR, where R for each occurrence is hydrogen, halogen, (C1-C4)alkyl, amino, or (C1-C4)alkylamino;
W is oxy, thio, amino, (C1-C4)alkylamino, or acetylamino;
Q is a heteroaryl group selected from the group consisting of pyridin-2-yl, pyridin-3-yl, furan-3-yl, uran-2-yl, thiophen-2-yl, thiophen-3-yl, thiazol-2-yl, pyrrol-2-yl, pyrrol-3-yl, pyrazol-3-yl, quinolin-2-yl, quinolin-3-yl, isoquinolin-3-yl, benzofuran-2-yl, benzofuran-3-yl, isobenzofuran-3-yl, benzothiophen-2-yl, benzothiophen-3-yl, indol-2-yl, indol-3-yl, 2H-imidazol-2-yl, oxazol-2-yl, isoxazol-3-yl, 1,2,4-oxadiazol-3-yl, 1,2,4-triazol-3-yl, and 1,2,4-oxathiazol-3-yl, where said heteroaryl group is optionally substituted with one to three substituents independently selected from halo, (C1-C4)alkyl or (C1-C4) alkyoxy;
R^{2a} and R^{2b} are each independently hydrogen, (C1-C4)alkyl, or partially or fully saturated (C3-C6) cycloalkyl;
R^{3a} and R^{3b} are each independently hydrogen, (C1-C4)alkyl, or (C1-C4)alkyl substituted with hydroxy, fluoro, or (C1-C4) alkoxy;
R⁴ is hydrogen, hydroxy, (C1-C4)alkyl, (C1-C4)alkyl substituted with hydroxy or cyano, (C1-C4)alkylcarbonyl, (C1-C4) alkoxy, (C1-C4)alkoxy-carbonyl, (C3-C4) alkenyl, or an amino-protecting group;
a nitrogen oxide thereof, a prodrug of the compound or the nitrogen oxide; a pharmaceutical acceptable salt of the compound, the nitrogen oxide, or the prodrug, or a solvate or hydrate of the compound, the nitrogen oxide, the prodrug, or the salt.

5. The use of claim 1, wherein the 5HT2C receptor agonist is a compound of formula (IIB): 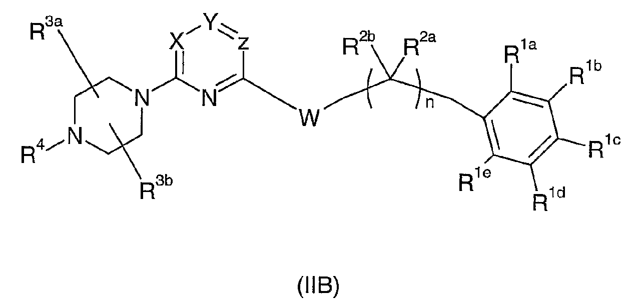 wherein:
Y is N; X and Z are each independently CR, where R for each occurrence is hydrogen, halogen (preferably Cl or F), (C1-C4)alkyl, amino, or (C1-C4)alkylamino;
W is oxy, thio, amino, (C1-C4)alkylamino, or acetylamino;
R^{1a}, R^{1b}, R^{1c}, R^{1d} and R^{1e} are each independently hydrogen, halogen, nitro, cyano, amino, (C1-C4)alkylamino, (C1-C4)alkyl, halo-substituted (C1-C4)alkyl, (C1-C4) alkoxy, halo-substituted (C1-C4) alkoxy, -C(O)NH², R^{1a} and R^{1b} taken together form a five-or six-membered, aromatic or partially or fully saturated fused ring, or R^{1a} taken together with R^{2a} or R^{2b} forms a five-or six-membered, fully saturated, fused ring;
R^{2a} and R^{2b} are each independently hydrogen, (C1-C4)alkyl, partially or fully saturated (C3-C6) cycloalkyl, or one of which taken together with Ria forms a five-or six-membered, fully saturated fused ring;
n is 0, 1, or 2;
R^{3a} and R^{3b} are each independently hydrogen, halogen, (C1-C4)alkyl, (C1-C4)alkyl substituted with hydroxy, fluoro, or (C1-C4) alkoxy;
R4 is hydrogen, hydroxy, (C1-C4)alkyl, (C1-C4)alkyl substituted with hydroxy or cyano, (C1-C4)alkylcarbonyl, (C1-C4)alkoxy, (C1-C4)alkoxy-carbonyl, or (C3-C4) alkenyl;
a nitrogen oxide thereof, a prodrug of the compound or the nitrogen oxide; a pharmaceutically acceptable salt of the compound, the nitrogen oxide, or the prodrug, or a solvate or hydrate of the compound, the nitrogen oxide, the prodrug, or the salt.

6. The use of claim 1, wherein the 5HT2C receptor agonist is a compound of formula (IIC): 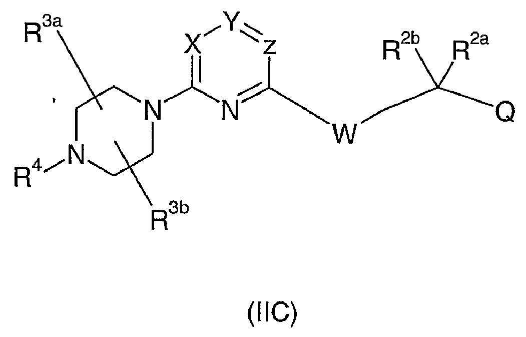 wherein:
Y is N; X and Z are each independently CR, where R for each occurrence is hydrogen, halogen, (C1-C4)alkyl, amino, or (C1-C4)alkylamino;
W is oxy, thio, amino, (C1-C4)alkylamino, or acetylamino;
Q is a heteroaryl group selected from the group consisting of pyridin-2-yl, pyridin-3-yl, furan-3-yl, furan-2-yl, thiophen-2-yl, thiophen-3-yl, thiazol-2-yl, pyrrol-2-yl, pyrrol-3-yl, pyrazol-3-yl, quinolin-2-yl, quinolin-3-yl, isoquinolin-3-yl, benzofuran-2-yl, benzofuran-3-yl, isobenzofuran-3-yl, benzothiophen-2-yl, benzothiophen-3-yl, indol-2-yl, indol-3-yl, 2H-imidazol-2-yl, oxazol-2-yl, isoxazol-3-yl, 1,2,4-oxadiazol-3-yl, 1,2,4-oxadiazol-5-yl, 1,3,4-oxadiazol-2-yl, 1,3,4-oxadiazol-5-yl, 1,2,4-triazol-3-yl, 1,2,3-thiadiazol-4-yl, 1,2,3-thiadiazol-5-yl, 1,3,4-thiadiazol-2-yl, 1,3,4-thiadiazol-5-yl, and 1,2,4-oxathiazol-3-yl, where the heteroaryl group is optionally substituted with one to three substituents independently selected from halo, (C1-C4)alkyl, cyano, nitro, amino, (C1-C4)alkylamino, or (C1-C4) alkyoxy;
R^{2a} and R^{2b} are each independently hydrogen, (C1-C4)alkyl, or partially or fully saturated (C3-C6) cycloalkyl;
R^{3a} and R^{3b} are each independently hydrogen, halogen, (C1-C4)alkyl, or (C1-C4)alkyl substituted with hydroxy, fluoro, or (C1-C4) alkoxy;
R⁴ is hydrogen, hydroxy, (C1-C4)alkyl, (C1-C4)alkyl substituted with hydroxy or cyano, (C1-C4)alkylcarbonyl, (C1-C4)alkoxy, (C1-C4)alkoxy-carbonyl, or (C3-C4) alkenyl;
a nitrogen oxide thereof, a prodrug of said compound or said nitrogen oxide; a pharmaceutically acceptable salt of said compound, said nitrogen oxide, or said prodrug, or a solvate or hydrate of said compound, said nitrogen oxide, said prodrug, or said salt.

7. The use of claim 1 wherein the EC₅₀ of the agonist for 5-HT2C receptor is less than 100 nM.

8. The use of claim 1 wherein the agonist for 5-HT2C receptor is selective for 5-HT2C receptors.

9. A method of screening for compounds useful for the treatment of stress urinary incontinence, comprising screening compounds for agonist activity against 5-HT2C receptor, and selecting compounds with an EC₅₀ of less than 100 nM.

10. Use of a compound in the manufacture of a medicament for the treatment of stress urinary incontinence, wherein said compound is identified by the method of claim 9.

11. A process for providing a medicament for the treatment of stress urinary incontinence, comprising the following steps:
(a) testing compounds in a ligand binding assay against 5-HT2C receptor;
(b) selecting a compound with an IC₅₀ of less than 100 nM;
(c) formulating a compound with the same structure as that selected in step (b), or a pharmaceutical acceptable salt thereof, with a pharmaceutically acceptable carrier or excipient.

12. A process for providing a medicament for the treatment of stress urinary incontinence, comprising the following steps:
(a) testing compounds in an assay, measuring the agonist-stimulated second messenger response of 5-HT2C receptors;
(b) selecting a compound with an EC₅₀ of less than 100 nM;
(c) formulating a compound with the same structure as that selected in step (b), with a pharmaceutically acceptable carrier or excipient.

13. The process of claim 11 or claim 12, additionally comprising the following steps:
(d) packaging the formulation of step (c);
(e) making the package of step (d) available to a patient suffering from stress urinary incontinence.

14. A process for preparing a medicament for the treatment of stress urinary incontinence, comprising the steps of (a) testing compounds in an assay suitable for detecting stimulation of 5-HT2C receptor, or testing compounds in an assay, measuring the agonist stimulated second messenger response of 5-HT2C receptor; (b) identifying one or more compounds capable of agonising 5-HT2C receptor with an EC₅₀ of less than 100 nM; and (c) preparing a quantity of those one or more identified compounds.

15. A method of preparing a composition for treating stress urinary incontinence which comprises:
(a) identifying a compound which specifically binds to 5-HT2C receptor by a method which comprises contacting cells expressing 5-HT2C receptor or membranes prepared from such cells with a radiolabelled 5-HT2C receptor ligand in the presence or absence of a test compound, measuring the radioactivity bound to the cells or membranes, comparing the radioactivity bound to the cells or membranes in the presence and absence of test compound, whereby a compound which causes a reduction in the radioactivity bound is a compound specifically binding to 5-HT2C receptor; and
(b) admixing said compound with a carrier.

16. A method of preparing a composition for treating stress urinary incontinence which comprises:
(a) identifying a compound which specifically activates 5-HT2C receptor by a method which comprises separately contacting cells expressing 5-HT2C receptor on their surface and producing a second messenger response in response to a 5-HT2C receptor agonist, or a membrane preparation of such cells, with the compound, under conditions suitable for activation of 5-HT2C receptor, and measuring the second messenger response, with an increase of the second messenger response after administration of the compound indicating that the compound is an agonist of the 5-HT2C receptor; and
(b) admixing said compound with a carrier.

17. The use, process, or method of any preceding claim, wherein said stress urinary incontinence is the stress component of mixed urinary incontinence.
